# EUROPEAN PATENT SPECIFICATION

(11) **EP 2 365 089 B1**
(45) Date of publication and mention of the grant of the patent: **14.05.2014**
(21) Application number: 10184678.0
(22) Date of filing: 17.03.2005
(51) Int. Cl.: C12P 21/00, C12R 1/645, C12N 1/14, C12N 15/52, C12N 5/10

(54) **Method of engineering a cytidine monophosphate-sialic acid synthetic pathway in yeast**
Verfahren zum Bau eines synthetischen Cytidinmonophosphat-Sialsäure-Pfads in Hefe
Procédé d'ingénierie d'une voie de synthèse de l'acide cytidine monophosphate-sialique chez les levures

(30) Priority: 17.03.2004 US 554139 P
(43) Date of publication of application: 14.09.2011
(62) Divisional of application: 05732104.4
(73) Proprietor: GlycoFi, Inc., Lebanon, NH 03766 (US)
(72) Inventor: Hamilton, Stephen R., Enfield, NH 03748 (US)
(74) Representative: Jaap, David Robert

(56) References cited:
- WO-A-00/52135
- WO-A-02/00879
- WO-A-96/21038
- WO-A-03/056914
- WO-A-2004/063370
- RODRIGUES MARCIO L ET AL: "Sialylglycoconjugates and sialyltransferase activity in the fungus Cryptococcus neoformans.", GLYCOCONJUGATE JOURNAL, vol. 19, no. 3, March 2002 (2002-03), pages 165-173, XP009053261, ISSN: 0282-0080
- AUMILLER JARED J ET AL: "A transgenic insect cell line engineered to produce CMP-sialic acid and sialylated glycoproteins", GLYCOBIOLOGY, IRL PRESS,, GB, vol. 13, no. 6, June 2003 (2003-06), pages 497-507, XP002320026, ISSN: 0959-6658
- GU XUEJUN ET AL: "Improvement of interferon-gamma sialylation in Chinese hamster ovary cell culture by feeding of N-acetylmannosamine", BIOTECHNOLOGY AND BIOENGINEERING, INTERSCIENCE PUBLISHERS, LONDON, GB, vol. 58, no. 6, 20 June 1998 (1998-06-20), pages 642-648, XP002171407, ISSN: 0006-3592
- BRETTHAUER R K ET AL: "GLYCOSYLATION OF PICHIA PASTORIS-DERIVED PROTEINS", BIOTECHNOLOGY AND APPLIED BIOCHEMISTRY, ACADEMIC PRESS, US, vol. 30, 1 January 1999 (1999-01-01), pages 193-200, XP002944839, ISSN: 0885-4513
- ALTMANN F ET AL: "INSECT CELLS AS HOSTS FOR THE EXPRESSION OF RECOMBINANT GLYCOPROTEINS", GLYCOCONJUGATE JOURNAL, CHAPMAN & HALL, GB, vol. 16, no. 2, 1 February 1999 (1999-02-01), pages 109-123, XP000990714, ISSN: 0282-0080, DOI: 10.1023/A:1026488408951

## Description

The present disclosure relates to the field of protein glycosylation. The present disclosure further relates to novel host cells comprising genes encoding activities in the cytidine monophosphate-sialic acid (CMP-Sia) pathway, which are particularly useful in the sialylation of glycoproteins in non-human host cells which lack endogenous CMP-Sia.

### BACKGROUND

Sialic acids (Sia) are a unique group of N- or O-substituted derivatives of N-acetylneuraminic acid (Neu5Ac) that are ubiquitous in animals of the deuterostome lineage, from starfish to humans. In other organisms, including most plants, protists, Archaea, and eubacteria, these compounds are thought to be absent (Warren, L. 1994). Exceptions have been identified, all of which are in pathogenic organisms, including certain bacteria, protozoa and fungi (Kelm, S. and Schauer, R. 1997) (Parodi, A.J. 1993) (Alviano, C.S., Travassos, L.R., et al. 1999). The mechanism by which pathogenic fungi, including *Cryptococcus neoformans* and *Candida albicans,* acquire sialic acid on cell surface glycoproteins and glycolipids remains undetermined (Alviano, C.S., Travassos, L.R., et al. 1999). It has been demonstrated, however, that when these organisms are grown in sialic acid-free media, sialic acid residues are found on cellular glycans, suggesting *de novo* synthesis of sialic acid. To date, no enzymes have been identified in fungi that are involved in the biosynthesis of sialic acid. The mechanism by which protozoa sialylate cell surface glycans has been well characterized. Protozoa, such as *Trypanosoma cruzi,* possess an external trans-sialidase that adds sialic acid to cell surface glycoproteins and glycolipids in a CMP-Sia independent mechanism (Parodi, A.J. 1993) The identification of a similar trans-sialidase in fungi would help to elucidate the mechanism of sialic acid transfer on cellular glycans, but such a protein has not yet been identified or isolated.

Despite the absence and/or ambiguity of sialic acid biosynthesis in fungi, sialic acid biosynthesis in pathogenic bacteria and mammalian cells is well understood. A group of pathogenic bacteria have been identified which possess the ability to synthesize sialic acids *de novo* to generate sialylated glycolipids that occur on the cell surface (Vimr, E., Steenbergen, S., et al. 1995). Although sialic acids on the surface of these pathogenic organisms are predominantly thought to be a means of evading the host immune system, it has been shown that these same sialic acid molecules are also involved in many processes in higher organisms, including protein targeting, cell-cell interaction, cell-substrate recognition and adhesion (Schauer, et al., 2000).

The presence of sialic acids can affect biological activity and *in vivo* half-life (MacDougall et all 1999). For example, the importance of sialic acids has been demonstrated in studies of the human erythropoietin (hEPO). The terminal sialic acid residues on the carbohydrate chains of the N-linked glycan of this glycoprotein prevent rapid clearance of hEPO from the blood and improve *in vivo* activity. Asialylated-hEPO (asialo-hEPO), which terminates in a galactose residue, has dramatically decreased erythropoietic activity *in vivo.* This decrease is caused by the increased clearance of the asialo-hEPO by the hepatic asialoglycoprotein receptor (Fukuda, M.N., Sasaki, H., et al. 1989) (Spivak, J.L. and Hogans, B.B. 1989). Similarly, the absence of the terminal sialic acid on many therapeutic glycoproteins can reduce efficacy, and thus require more frequent dosing.

Although many of the currently available therapeutic glycoproteins are made in mammalian cell lines, these systems are expensive and typically yield low product titers. To overcome these shortcomings the pharmaceutical industry is currently investigating new approaches. One approach is the production of glycoproteins in fungal systems. Fungal expression systems are less expensive to maintain, and are capable of producing higher titers per unit culture (Cregg, J.M. et al., 2000). The disadvantage, however, is that fungal and mammalian glycosylation differ greatly, and therapeutic proteins with non-human glycosylation have a high risk of eliciting an immune response in humans (Ballou, C.E., 1990). Although the initial stages of N-linked glycosylation in the endoplasmic reticulum are similar in fungi and mammals, subsequent processing in the Golgi results in dramatically different glycans. Nonetheless, these divergent glycosylation pathways can be overcome by genetically engineering the fungal host to produce human-like glycoproteins as described in WO 02/00879, WO 031056914, US 2004/0018590, Choi et al., 2003 and Hamilton et al., 2003. It is, therefore, desirable to have a novel protein expression system (e.g., fungal system) that is capable of producing fully sialylated human-like glycoproteins.

A method to engineer a CMP-Sia biosynthetic pathway into non-human host cells which lack endogenous CMP-Sia is needed. Non-human hosts which lack endogenous CMP-Sia include most lower eukaryotes such as fungi, most plants and non-pathogenic bacteria,

To date, no fungal system has been identified that generates sialylated glycoproteins from an endogenous pool of the sugar substrate CMP-Sia. What is needed, therefore, is a method to engineer a CMP-Sia biosynthetic pathway into a non-human host which lacks endogenous CMP-Sia, such as a fungal host, to ensure that substrates required for sialylation are present in useful quantities for the production of therapeutic glycoproteins.

### SUMMARY

The invention is defined by the embodiments of the claims. The invention relates to a method for producing CMP-sialic acid in a yeast host cell comprising (a) transforming into the yeast host cell nucleic acid molecules encoding the enzymes of a sialyation pathway, which enzymes consist of *E. coli* NeuC, *E. coli* NeuB and a CMP-sialic acid synthase; and (b) growing the yeast host cell under conditions effective for producing the CMP-sialic acid. The invention further relates to a yeast host cell capable of producing CMP-sialic acid comprising a sialyation pathway consisting of *E. coli* NeuC, *E. coli* NeuB and a CMP-sialic acid synthase, wherein the yeast host cell is capable of producing the CMP-sialic acid. The invention yet further relates to a method for producing CMP-sialic acid comprising (a) providing a yeast host comprising a CMP-sialic acid biosynthetic pathway consisting of *E. coli* NeuC, *E. coli* NeuB, and a CMP-sialic acid synthase; and (b) growing the yeast host cell under conditions effective for producing the CMP-sialic acid.

A method for engineering a functional CMP-sialic acid (CMP-Sia) biosynthetic pathway into a non-human host cell lacking endogenous CMP-Sia, such as a fungal host cell, is provided. The method involves the cloning and expression of several enzymes of mammalian origin, bacterial origin or both, in a host cell, particularly a fungal host cell. The engineered CMP-Sia biosynthetic pathway is useful for producing sialylated glycolipids, O-glycans and N-glycans *in vivo.* The present disclosure is thus useful for facilitating the generation of sialylated therapeutic glycoproteins in non-human host cells lacking endogenous sialylation, such as fungal host cells.

### Modified Hosts Comprising A Cellular Pool Of CMP-Sia Or A CMP-Sia Biosynthetic Pathway

The invention comprises a recombinant non-human host cell comprising a cellular pool of CMP-Sia, wherein the host cell lacks endogenous CMP-Sia. In one embodiment, the CMP-Sia comprises a sialic acid selected from Neu5Ac, N-glycolylneuraminic acid (Neu5Gc), and keto-3-deoxy-D-glycero-D-galacto-nononic acid (KDN).

The invention further comprises a recombinant non-human host cell comprising a CMP-Sia biosynthetic pathway, wherein the host cell lacks endogenous CMP-Sia.

In another embodiment, the invention comprises a non-human host cell comprising recombinant enzymes that participate in the biosynthesis of CMP-Sia; wherein the host cell lacks endogenous CMP-Sia.

In one embodiment, the host cell is a fungal host cell.

In one embodiment, the host cell of the invention produces at least one intermediate selected from the group consisting of UDP-GlcNAc, ManNAc, ManNAc-6-P, Sia-9-P and Sia. In one embodiment, the intermediate is UDP-GlcNAc. In one embodiment, the intermediate is ManNAc. In one embodiment, the intermediate is ManNAc-6-P. In one embodiment, the intermediate is Sia-9-P. In one embodiment, the intermediate is Sia.

In one embodiment, the host cell of the invention comprises a cellular pool of CMP-Sia. In one embodiment, the CMP-Sia comprises a sialic acid selected from NeuSAc, N-glycolylneuraminic acid (Neu5Gc), and keto-3-deoxy-D-glycero-D-galacto-nononic acid (KDN).

In one embodiment, the host cell of the invention expresses one or more enzyme activities selected from *E*. *coli NeuC, E. coli NeuB* and *E. coli NeuA.*

In one embodiment, the host cell expresses one or more enzyme activities selected from *E*. *coli NeuC, E. coli NeuB* and a mammalian CMP-sialate syathase activity.

In one embodiment, the host cell expresses one or more enzyme activities selected from *E*. *coli NeuC, E. coli NeuB* and a mammalian CMP-sialate synthase activity, and further expresses at least one enzyme activity selected from UDP-GlcNAc epimerase, sialate synthase, CMP-sialate synthase, UDP-N-acetylglucosamine-2-epimerase, N-acetylmannosamine kinase, N-acetyl-neuraminate-9-phosphate synthase, N-acetylneuraminate-9-phosphatase and CMP-sialic acid synthase.

In one embodiment, the host cell expresses at least one enzyme activity selected from UDP-GlcNAc epimerase, sialate synthase, CMP-sialate synthase, UDP-N-acetylglucosamine-2-epimerase, N-acetylmannosamine kinase, N-acetylneuraminate-9-phosphate synthase, N-acetylneuraminate-9-phosphatase and CMP-sialic acid synthase.

In one embodiment, the host cell of the invention empresses *E. coli NeuC.* In one embodiment, the host cell expresses *E. coli NeuB.* In one embodiment, the host cell expresses *E. coli NeuA.*

In one embodiment, the host cell of the invention expresses the enzyme activity of UDP-GlcNAc epimerase. In one embodiment, the host cell of the invention expresses the enzyme activity of sialate synthase. In one embodiment, the host cell of the invention expresses the enzyme activity of CMP-sialate synthase. In one embodiment, the host cell expresses the enzyme activity of UDP-N-acetylglucosamine-2-epimerase. In one embodiment, the host cell expresses the enzyme activity of N-acetylmannosamine kinase. In one embodiment, the host cell expresses the enzyme activity of N-acetylneuraminate-9-phosphate synthase. In one embodiment, the host cell expresses the enzyme activity ofN-acetylneuraminate-9-phosphatase. In one embodiment, the host cell disclosed herein expresses the enzyme activity of CMP-sialic acid synthase.

In one embodiment, the enzyme activity of *NeuC* is expressed from a nucleic acid comprising the nucleic acid sequence of SEQ ID NO:13, or a portion thereof. In one embodiment, the enzyme acivity of *NeuC* is from a poplypeptide comprising the amino acid sequence of SEQ ID NO:14 or a fragment thereof.

In one embodiment, the enzyme activity of *NeuB* is expressed from a nucleic acid comprising the nucleic acid sequence of SEQ ID NO:15, or a portion thereof. In one embodiment, the enzyme acivity of *NeuB* is from a poplypeptide comprising the amino acid sequence of SEQ ID NQ:16 or a fragment thereof.

In one embodiment, the enzyme activity of *NeuA* is expressed from a nucleic acid comprising the nucleic acid sequence of SEQ ID NO:17, or a portion thereof. In one embodiment, the enzyme acivity of *NeuA* is from a poplypeptide comprising the amino acid sequence of SEQ ID NO: 18 or a fragment thereof

In one embodiment, the enzyme activity of CMP-synthase is expressed from a nucleic acid comprising the nucleic acid sequence of SEQ ID NO:19, or a portion thereof In one embodiment, the enzyme acivity of CMP-synthase is from a poplypeptide comprising the amino acid sequence of SEQ UD NO:20 or a fragment thereof.

In one embodiment, the enzyme activity of CMP-synthase is expressed from a nucleic acid comprising the nucleic acid sequence of GenBank Accession No. AF397212, or a portion thereof. In one embodiment, the enzyme acivity of CMP-synthase is from a poplypeptide comprising the amino acid sequence of AAM90588 or a fragment thereof.

In one embodiment, the enzyme activity of GlcNAc epimerase is expressed from a nucleic acid comprising the nucleic acid sequence of SEQ ID NO:21, or a portion thereof In one embodiment, the enzyme acivity of GlcNAc is from a poplypeptide comprising the amino acid sequence of SEQ ID NO:22 or a fragment thereof.

In one embodiment, the enzyme activity of sialate aldolase is expressed from a nucleic acid comprising the nucleic acid sequence of SEQ ID NO:23, or a portion thereof. In one embodiment, the enzyme acivity of sialate aldolase is from a poplypeptide comprising the amino acid sequence of SEQ ID NO:24 or a fragment thereof.

In one embodiment, the host cell disclosed herein produces at least one intermediate selected from the group consisting of UDP-GlcNAc, ManNAc, ManNAc-6-P, Sia-9-P and Sia. In one embodiment, the intermediate is UDP-GlcNAc. In one embodiment, the intermediate is ManNAc. In one embodiment, the intermediate is ManNAc-6-P. In one embodiment, the intermediate is Sia-9-P. In one embodiment, the intermediate is Sia.

In one embodiment, the host cell disclosed herein expresses a heterologous therapeutic protein. In one embodiment, said therapeutic protein is selected from the group consisting of: erythropoietin, cytokines, interferon-α, interferon-β, interferon-γ, interferon-ω, TNF-α, granulocyte-CSF, GM-CSF, interleukins, IL-1ra, coagulation factors, factor VIII, factor IX, human protein C, antithrombin III and thrombopoeitin, IgA antibodies or fragments thereof, IgG antibodies or fragments thereof, IgA antibodies or fragments thereof, IgD antibodies or fragments thereof, IgE antibodies or fragments thereof, IgM antibodies and fragments thereof, soluble IgE receptor α-chain urokinase, chymase, urea trypsin inhibitor, IGF-binding protein, epidermal growth factor, growth hormone-releasing factor, FSH, annexin V fusion protein, angiostatin, vascular endothelial growth factor-2, myeloid progenitor inhibitory factor-1, osteoprotegerin, α-1 antitrypsin, DNase II, α-feto proteins and glucocerebrosidase.

In one embodiment, the host cell is from a fungal host. In one embodiment, the fungal host is selected from the group consisting of *Pichia pastoris, Pichia finlandica, Pichia trehalophila, Pichia koclamae, Pichia membranaefaciens, Pichia minuta, Ogataea minuta, Pichia lindneri, Pichia opuntiae, Pichia thermotolerans, Pichia salictaria, Pichia guercuum, Pichia pijperi, Pichia stiptis, Pichia methanolica, Pichia sp., Saccharomyces cerevisiae, Saccharomyces sp., Hansenula polymorpha, Kluyveromyces sp., Kluyveromyces lactis, Candida albicans, Aspergillus nidulans, Aspergillus niger, Aspergillus oryzae, Aspergillus sp, Trichoderma reesei, Chrysosporium lucknowense, Fusarium sp., Fusarium gramineum, Fusarium venenatum* and *Neurospora crassa.* In one embodiment, the fungal host is *P. pastoris.*

In one embodiment, the host cell is from a non-pathogenic bacteria. In another emobidment, the host cell is from a plant.

In one embodiment, the enzyme activity is expressed under the control of a constitutive promoter.

In another embodiment, the enzyme activity is expressed under the control of an inducible promoter.

In one embodiment, the expressed enzyme activity is from a partial ORF encoding that enzymatic activity.

In another embodiment, the expressed enzyme is a fusion to another protein or peptide.

In another embodiment, the expressed enzyme has been mutated to enhance or attenuate the enzymatic activity.

In one embodiment, the recombinant host cells disclosed herein have modified oligosaccharides which may be modified further by heterologous expression of a set of glycosyltransferases, sugar transporters and mannosides as described in WO02/00879, WO03/056914 and US 2004/0018590.

### Method Of Producing CMP-Sia In A Host

The invention further comprises a method for producing CMP-Sia in a recombinant non-human host comprising expressing a CMP-Sia biosynthetic pathway.

In one embodiment, the invention comprises a method for producing CMP-Sia, comprising expressing in a non-human host cell recombinant enzymes that participate in the biosynthesis of CMP-Sia.

In one embodiment, the host cell disclosed herein is a fungal host cell.

In one embodiment, the method disclosed herein comprises expressing at least one enzyme activity from a prokaryotic CMP-Sia biosynthetic pathway. In one embodiment, the method of the invention comprises expressing the enzyme activity consisting *of E. coli NeuC, E. coli NeuB* and *E. col. NeuA* activity.

In another embodiment, the method disclosed herein comprises expressing at least one enzyme activity from a mammalian CMP-Sia biosynthetic pathway.

In one embodiment, the method disclosed herein comprises expressing a mammalian CMP-sialate synthase activity. In one embodiment, the CMP-sialate synthase activity localizes in the nucleus.

In one embodiment, the method disclosed herein comprises expressing a hybrid CMP-Sia biosynthetic pathway. In one embodiment, the method disclosed herein comprises expressing at least one enzyme activity selected from *E. coli NeuC, E. coli NeuB* and a mammalian CMP-sialate synthase activity. In one embodiment, the CMP-sialate synthase activity localizes in the nucleus.

In one embodiment, the enzyme activity *of NeuB* is expressed from a nucleic acid comprising the nucleic acid sequence of SEQ ID NO: 15, or a portion thereof. In one embodiment, the enzyme acivity of *NeuB* is from a poplypeptide comprising the amino acid sequence of SEQ ID NO: 16 or a fragment thereof.

In one embodiment, the enzyme activity *of NeuA* is expressed from a nucleic acid comprising the nucleic acid sequence of SEQ ID NO:17, or a portion thereof. In one embodiment, the enzyme acivity *of NeuA* is from a poplypeptide comprising the amino acid sequence of SEQ ID NO: 18 or a fragment thereof.

In one embodiment, the enzyme activity of CMP-synthase is expressed from a nucleic acid comprising the nucleic acid sequence of SEQ ID NO:19, or a portion thereof. In one embodiment, the enzyme acivity of CMP-synthase is from a poplypeptide comprising the amino acid sequence of SEQ UD NO:20 or a fragment thereof

In one embodiment, the enzyme activity of CMP-synthase is expressed from a nucleic acid comprising the nucleic acid sequence of GenBank Accession No. AF397212, or a portion thereof. In one embodiment, the enzyme acivity of CMP-synthase is from a poplypeptide comprising the amino acid sequence of AAM90588 or a fragment thereof.

In one embodiment, the method disclosed herein comprises using a host cell which expresses a heterologous therapeutic protein. In one embodiment, said therapeutic protein is selected from the group consisting of: erythropoietin, cytokines, interferon-α, interferon-β, interferon-γ, interferon-ω, TNF-α, granulocyte-CSF, GM-CSF, interleukins, IL-1ra, coagulation factors, factor VIII, factor IX, human protein C, antithrombin III and thrombopoeitin, IgA antibodies or fragments thereof, IgG antibodies or fragments thereof, IgA antibodies or fragments thereof, IgD antibodies or fragments thereof, IgE antibodies or fragments thereof, IgM antibodies and fragments thereof, soluble IgE receptor α-chain, urokinase, chymase, urea trypsin inhibitor, IGF-binding protein, epidermal growth factor, growth hormone-releasing factor, FSH, annexin V fusion protein, angiostatin, vascular endothelial growth factor-2, myeloid progenitor inhibitory factor-1, osteoprotegerin, α-1 antitrypsin, DNase II, α-feto proteins and glucocerebrosidase.

In one embodiment, the non-human host cell to be used is from a fungal host. In one embodiment, the fungal host is selected from the group consisting of *Pichiapastoris, Pichia finlandica, Pichia trehalophila, Pichia koclamae, Pichia membranaefaciens, Pichia minuta (Ogataea minuta, Pichia lindneri), Pichia opuntiae, Pichia thermotolerans, Pichia salictaria, Pichia guercuum, Pichia pijperi, Pichia stiptis, Pichia methanolica, Pichia sp., Saccharomyces cerevisiae, Saccharomyces sp., Hansenula polymorpha, Kluyveromyces sp., Kluyveromyces lactis, Candida albicans, Aspergillus nidulans, Aspergillus niger, Aspergillus oryzae, Aspergillus sp, Trichoderma reesei, Chrysosporium lucknowense, Fusarium sp., Fusarium gramineum, Fusariwn venenatum* and *Neurospora crassa.* In one embodiment, the fungal host is *Pichia pastoris.*

In one embodiment, the host cell dislosed herein is from a non-pathogenic bacteria. In another emobidment, the host cell disposed herein is from a plant

In one embodiment, the CMP-Sia synthesis is enhanced by supplementing a medium for growing the non-human host cell with one or more intermediate substrates used in the CMP-Sia synthesis. In one embodiment, the intermediates are selected from the group consisting of UDP-GlcNAc, ManNAc, ManNAc-6-P, Sia-9-P and Sia.

In one embodiment, the enzyme activity is expressed under the control of a constitutive promoter.

In another embodiment, the enzyme activity is expressed under the control of an inducible promoter.

In one embodiment, the expressed enzyme activity is from a partial ORF encoding that enzymatic activity.

In another embodiment, the expressed enzyme is a fusion to another protein or peptide.

In another embodiment, the expressed enzyme has been mutated to enhance or attenuate the enzymatic activity.

In one embodiment the methods described above comprise the use of a host having modified oligosaccharides which may be modified further by heterologous expression of a set of glycosyltransferases, sugar transporters and mannosides as described in WO02/00879, W003/056914 and US 2004/0018590.

### Methods Of Producing Recombinant Glycoproteins

In one embodiment, the disclosure provides a method for producing recombinant glycoprotein comprising the step of producing a cellular pool of CMP-Sia in a recombinant non-human host cell which lacks endogenous CMP-Sia and expressing the glycoprotein in said host. In one embodiment, the host is a fungal host.

In another embodiment, the disclosure provides a method for producing recombinant glycoprotein comprising the step of engineering a CMP-Sia biosynthetic pathway in a non-human host cell which lacks endogenous CMP-Sia and expressing the glycoprotein said host In one embodiment, the host is a fungal host In one embodiment, the CMP-Sia pathway results in the formation of a cellular pool of CMP-Sia.

In another embodiment, the disclosure provides a method for producing recombinant glycoprotein comprising the step of expressing one or more recombinant enzymes that participate in the biosynthesis of CMP-Sia in a non-human host cell which lacks endogenous CMP-Sia and expressing the glycoprotein in said host. In one embodiment, the host is a fungal host

In any of the embodiments disclosad herein, the recombinant non-human host cell may have modified oligosaccharides which may be modified further by heterologous expression of recombinant glycosylation enzymes (such as sialyltransferases, mannosidases, fucosyltransferases, galactosyltransferases, GclNAc transferases, ER and Golgi specific transporters, enzymes involved in the processing of oligosaccharides, and enzymes involved in the synthesis of activated oligosaccharide precursors such as UDP-galactose and CMP-N-acetylneuraminic acid) which may be necessary for the production of a human-like glycoprotein in a non-human host as described in WO02/00879, W003/056914 and US 2004/0018590.

In any of the embodiments disclosed herein, the host cell may express a heterologous therapeutic protein. In one embodiment, said therapeutic protein is selected from the group consisting of: erythropoietin, cytokines, interferon-α, interferon-β, interferon-γ interferon-ω, TNF-α, granulocyte-CSF, GM-CSF, interleukins, IL-1ra, coagulation factors, factor VIII, factor IX, human protein C, antithrombin III and thrombopoeitin, IgA antibodies or fragments thereof, IgG antibodies or fragments thereof, IgA antibodies or fragments thereof, IgD antibodies or fragments thereof, IgE antibodies or fragments thereof, IgM antibodies and fragments thereof, soluble IgE receptor α-chain, urokinase, chymase, urea trypsin inhibitor, IGF-binding protein, epidermal growth factor, growth hormone-releasing factor, FSH, annexin V fusion protein, angiostatin, vascular endothelial growth factor-2, myeloid progenitor inhibitory factor-1, osteoprotegerin, α-1 antitrypsin, DNase II, α-feto proteins and glucocerebrosidase.

It is to be understood that single or multiple enzymatic activities may be introduced into a non-human host cell in any fashion, by use of one or more nucleic acid molecules, without necessarily using a nucleic acid, plasmid or vector that is specifically disclosed in the foregoing description.

### BRIEF DESCRIPTION OF THE DRAWINGS

**Figure 1** illustrated the CMP-sialic acid biosynthetic pathway in mammals and bacteria. Enzymes involved in each pathway are italicized. The primary substrates, intermediates and products are in bold. (PEP: phosphoenol pyruvate; CTP: cytidine triphosphate).
**Figure 2** shows the open reading frame (ORF) *of E. coli* protein *NeuC* (Genbank: M84026.1; SEQ ID NO: 13) and the predicted amino acid sequence (SEQ ID NO: 14). The underlined DNA sequences are regions to which primers have been designed to amplify the ORF.
**Figure 3** shows the ORF of *E. coli* protein *NeuB* (Genbank: U05248.1; SEQ ID NO: 15) and the predicted amino acid sequence (SEQ ID NO:16). The underlined DNA sequences are regions to which primers have been designed to amplify the ORF.
**Figure 4** shows the ORF of *E. coli* protein *NeuA* (Genbank: J05023.1; SEQ ID NO:17) and the predicted amino acid sequence (SEQ ID NO:18). The underlined DNA sequences are regions to which primers have been designed to amplify the ORF.
**Figure 5** shows the ORF of *Mus musculus* CMP-Sia synthase (Genbank: AJ006215; SEQ ID NO:19) and the amino acid sequence (SEQ ID NO:20). The underlined DNA sequences are regions to which primers have been designed to amplify the ORF.
**Figure 6** illustrates an alternative biosynthetic route for generating *N-*acetylmannosamine (ManNAc) *in vivo.* Enzymes involved in each pathway are italicized. The primary substrates, intermediates and products are in bold.
**Figure 7** shows the ORF of *Sus scrofa* GlcNAc epimerase (Genbank: D83766; SEQ ID NO: 21) and the amino acid sequence (SEQ ID NO:22). The underlined DNA sequences are regions to which primers have been designed to amplify the ORF.
**Figure 8** illustrates the reversible reaction catalyzed by sialate aldolase and its dependence on sialic acid (Sia) concentration. Enzymes involved in each pathway are italicized. The primary substrates, intermediates and products are in bold.
**Figure 9** shows the ORF of *E. coli* sialate aldolase (Genbank: X03345; SEQ ID NQ:23) and the amino acid sequence (SEQ ID NO:24). The underlined DNA sequences are regions to which primers have been designed to amplify the ORF.
**Figure 10** shows a HPLC of negative control of cell extracts from strain YSH99a incubated under assay conditions (**Example 10**) in the absence of acceptor glycan. The doublet peak eluting at 26.5 min results from contaminating cellular component(s).
**Figure 11** shows a HPLC of positive control cell extract from strain YSH99a incubated under assay conditions (**Example 10**) in the presence of 2-AB (aminobenzamide) labeled acceptor glycan and supplemented with CMP-sialic acid. The peak eluting at 23 min corresponds to sialylation on each branch of a biantennary galactosylated N-glycan. The doublet peak eluting at 26.5 min results from contaminating cellular component(s).
**Figure 12** shows a HPLC of a cell extract from strain YSH99a incubated under assay conditions (**Example 10**) in the presence of acceptor glycan with no exogenous CMP-sialic acid. The peaks eluting at 20 and 23 min correspond to mono- and di-sialylation of a biantennary galactosylated N-glycan. The doublet peak eluting at 26.5 min results from contaminating cellular component(s).
**Figure 13** shows sialidase treatment ofN-glycans from YSH99a extract incubation. The sample illustrated in **Figure 12** was incubated overnight at 37°C in the presence of 100 U sialidase (New England Biolabs, Beverley, MA). The peaks eluting at 20 and 23 min, corresponding to mono- and di-sialylated N-glycan, have been removed. The contaminating peak at 26 min remains.
**Figure 14** shows commercial mono- and di-sialylated N-glycan standards. The peaks eluting at 20 and 23 min correspond to mono- and di-siaiylation of the commercial standards A1 and A2 (Glyko Inc., San Rafael, CA).

### DETAILED DESCRIPTION

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. The methods and techniques disclosed herein are generally performed according to conventional methods well known in the art. Generally, nomenclatures used in connection with, and techniques of biochemistry, enzymology, molecular and cellular biology, microbiology, genetics and protein and nucleic acid chemistry and hybridization described herein are those well known and commonly used in the art. The methods and techniques disclosed herein are generally performed according to conventional methods well known in the art and as described in various general and more specific references that are cited and discussed throughout the present specification unless otherwise indicated. See, e.g., Sambrook, J. and Russell, D.W. (2001); Ausubel et al., Current Protocols in Molecular Biology, Greene Publishing Associates (1992, and Supplements to 2002); Harlow and Lane Antibodies: A Laboratory Manual, Cold Spring Harbor Laboratory Press, Cold Spring Harbor, N.Y. (1990); Introduction to Glycobiology, Maureen E. Taylor, Kurt Drickamer, Oxford Univ. Press (2003); Worthington Enzyme Manual, Worthington Biochemical Corp. Freehold, NJ; Handbook of Biochemistry: Section A Proteins Vol I 1976 CRC Press; Handbook of Biochemistry: Section A Proteins Vol II 1976 CRC Press; Essentials of Glycobiology, Cold Spring Harbor Laboratory Press (1999). The nomenclatures used in connection with, and the laboratory procedures and techniques of, biochemistry and molecular biology described herein are those well known and commonly used in the art.

The following terms, unless otherwise indicated, shall be understood to have the following meanings:

The term "polynucleotide" or "nucleic acid molecule" refers to a polymeric form of nucleotides of at least 10 bases in length. The term includes DNA molecules (e.g., cDNA or genomic or synthetic DNA) and RNA molecules (e.g., mRNA or synthetic RNA), as well as analogs of DNA or RNA containing non-natural nucleotide analogs, non-native internucleoside bonds, or both. The nucleic acid can be in any topological conformation. For instance, the nucleic acid can be single-stranded, double-stranded, triple-stranded, quadruplexed, partially double-stranded, branched, hairpinned, circular, or in a padlocked conformation. The term includes single and double stranded forms of DNA.

Unless otherwise indicated, a "nucleic acid comprising SEQ ID NO:X" refers to a nucleic acid, at least a portion of which has either (i) the sequence of SEQ ID NO:X, or (ii) a sequence complementary to SEQ ID NO:X. The choice between the two is dictated by the context. For instance, if the nucleic acid is used as a probe, the choice between the two is dictated by the requirement that the probe be complementary to the desired target.

An "isolated" or "substantially pure" nucleic acid or polynucleotide (e.g., an RNA, DNA or a mixed polymer) is one which is substantially separated from other cellular components that naturally accompany the native polynucleotide in its natural host cell, e.g., ribosomes, polymerases, and genomic sequences with which it is naturally associated. The term embraces a nucleic acid or polynucleotide that (1) has been removed from its naturally occurring environment, (2) is not associated with all or a portion of a polynucleotide in which the "isolated polynucleotide" is found in nature, (3) is operatively linked to a polynucleotide which it is not linked to in nature, or (4) does not occur in nature. The term "isolated" or "substantially pure" also can be used in reference to recombinant or cloned DNA isolates, chemically synthesized polynucleotide analogs, or polynucleotide analogs that are biologically synthesized by heterologous systems.

However, "isolated" does not necessarily require that the nucleic acid or polynucleotide so described has itself been physically removed from its native environment. For instance, an endogenous nucleic acid sequence in the genome of an organism is deemed "isolated" herein if a heterologous sequence (i.e., a sequence that is not naturally adjacent to this endogenous nucleic acid sequence) is placed adjacent to the endogenous nucleic acid sequence, such that the expression of this endogenous nucleic acid sequence is altered. By way of example, a non-native promoter sequence can be substituted (e.g., by homologous recombination) for the native promoter of a gene in the genome of a human cell, such that this gene has an altered expression pattern. This gene would now become "isolated" because it is separated from at least some of the sequences that naturally flank it.

A nucleic acid is also considered "isolated" if it contains any modifications that do not naturally occur to the corresponding nucleic acid in a genome. For instance, an endogenous coding sequence is considered "isolated" if it contains an insertion, deletion or a point mutation introduced artificially, e.g., by human intervention. An "isolated nucleic acid" also includes a nucleic acid integrated into a host cell chromosome at a heterologous site, a nucleic acid construct present as an episome. Moreover, an "isolated nucleic acid" can be substantially free of other cellular material, or substantially free of culture medium when produced by recombinant techniques, or substantially free of chemical precursors or other chemicals when chemically synthesized.

As used herein, the phrase "degenerate variant" of a reference nucleic acid sequence encompasses nucleic acid sequences that can be translated, according to the standard genetic code, to provide an amino acid sequence identical to that translated from the reference nucleic acid sequence.

The term "percent sequence identity" or "identical" in the context of nucleic acid sequences refers to the residues in the two sequences which are the same when aligned for maximum correspondence. The length of sequence identity comparison may be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides. There are a number of different algorithms known in the art which can be used to measure nucleotide sequence identity. For instance, polynucleotide sequences can be compared using FASTA, Gap or Bestfit, which are programs in Wisconsin Package Version 10.0, Genetics Computer Group (GCG), Madison, Wisconsin. FASTA provides alignments and percent sequence identity of the regions of the best overlap between the query and search sequences (Pearson, 1990, (herein incorporated by reference). For instance, percent sequence identity between nucleic acid sequences can be determined using FASTA with its default parameters (a word size of 6 and the NOPAM factor for the scoring matrix) or using Gap with its default parameters as provided in GCG Version 6.1, herein incorporated by reference.

The term "substantial homology" or "substantial similarity," when referring to a nucleic acid or fragment thereof, indicates that, when optimally aligned with appropriate nucleotide insertions or deletions with another nucleic acid (or its complementary strand), there is nucleotide sequence identity in at least about 50%, more preferably 60% of the nucleotide bases, usually at least about 70%, more usually at least about 80%, preferably at least about 90%, and more preferably at least about 95%, 96%, 97%, 98% or 99% of the nucleotide bases, as measured by any well-known algorithm of sequence identity, such as FASTA, BLAST or Gap, as discussed above.

Alternatively, substantial homology or similarity exists when a nucleic acid or fragment thereof hybridizes to another nucleic acid, to a strand of another nucleic acid, or to the complementary strand thereof, under stringent hybridization conditions. "Stringent hybridization conditions" and "stringent wash conditions" in the context of nucleic acid hybridization experiments depend upon a number of different physical parameters. Nucleic acid hybridization will be affected by such conditions as salt concentration, temperature, solvents, the base composition of the hybridizing species, length of the complementary regions, and the number of nucleotide base mismatches between the hybridizing nucleic acids, as will be readily appreciated by those skilled in the art One having ordinary skill in the art knows how to vary these parameters to achieve a particular stringency of hybridization.

In general, "stringent hybridization" is performed at about 25°C below the thermal melting point (Tₘ) for the specific DNA hybrid under a particular set of conditions. "Stringent washing" is performed at temperatures about 5°C lower than the Tₘ for the specific DNA hybrid under a particular set of conditions. The Tₘ is the temperature at which 50% of the target sequence hybridizes to a perfectly matched probe. See Sambrook, J. and Russell, D.W. (2001), *supra,* page 9.51.

For purposes herein, "high stringency conditions" are defined for solution phase hybridization as aqueous hybridization (i.e., free of formamide) in 6X SSC (where 20X SSC contains 3.0 M NaCl and 0.3 M sodium citrate), 1% SDS at 65°C for 8-12 hours, followed by two washes in 0.2X SSC, 0.1% SDS at 65°C for 20 minutes. It will be appreciated by the skilled worker that hybridization at 65°C will occur at different rates depending on a number of factors including the length and percent identity of the sequences which are hybridizing.

The nucleic acids (also referred to as polynucleotides) disclosed herein may include both sense and antisense strands of RNA, cDNA, genomic DNA, and synthetic forms and mixed polymers of the above. They may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (e.g., methyl phosphonates, phosphotriesters, phosphoramidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.). Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

The term "mutated" when applied to nucleic acid sequences means that nucleotides in a nucleic acid sequence may be inserted, deleted or changed compared to a reference nucleic acid sequence. A single alteration may be made at a locus (a point mutation) or multiple nucleotides may be inserted, deleted or changed at a single locus. In addition, one or more alterations may be made at any number of loci within a nucleic acid sequence. A nucleic acid sequence may be mutated by any method known in the art including but not limited to mutagenesis techniques such as "error-prone PCR" (a process for performing PCR under conditions where the copying fidelity of the DNA polymerase is low, such that a high rate of point mutations is obtained along the entire length of the PCR product. See, e.g., Leung, D.W., et al., Technique, 1, pp. 11-15 (1989) and Caldwell, R.C. & Joyce G.F., PCR Methods Applic., 2, pp. 28-33 (1992)); and "oligonucleotide-directed mutagenesis" (a process which enables the generation of site-specific mutations in any cloned DNA segment of interest. See, e.g., Reidhaar-Olson, J.F. & Sauer, R.T., et al., Science 241, pp. 53-57 (1988)).

The term "vector" as used herein is intended to refer to a nucleic acid molecule capable of transporting another nucleic acid to which it has been linked. One type of vector is a "plasmid", which refers to a circular double stranded DNA loop into which additional DNA segments may be ligated. Other vectors include cosmids, bacterial artificial chromosomes (BAC) and yeast artificial chromosomes (YAC). Another type of vector is a viral vector, wherein additional DNA segments may be ligated into the viral genome (discussed in more detail below). Certain vectors are capable of autonomous replication in a host cell into which they are introduced (e.g., vectors having an origin of replication which functions in the host cell). Other vectors can be integrated into the genome of a host cell upon introduction into the host cell, and are thereby replicated along with the host genome. Moreover, certain preferred vectors are capable of directing the expression of genes to which they are operatively linked. Such vectors are referred to herein as "recombinant expression vectors" (or simply, "expression vectors").

"Operatively linked" expression control sequences refers to a linkage in which the expression control sequence is contiguous with the gene of interest to control the gene of interest, as well as expression control sequences that act in *trans* or at a distance to control the gene of interest.

The term "expression control sequence" as used herein refers to polynucleotide sequences which are necessary to affect the expression of coding sequences to which they are operatively linked. Expression control sequences are sequences which control the transcription, post-transcriptional events and translation of nucleic acid sequences. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (e.g., ribosome binding sites); sequences that enhance protein stability; and when desired, sequences that enhance protein secretion. The nature of such control sequences differs depending upon the host organism; in prokaryotes, such control sequences generally include promoter, ribosomal binding site, and transcription termination sequence. The term "control sequences" is intended to include, at a minimum, all components whose presence is essential for expression, and can also include additional components whose presence is advantageous, for example, leader sequences and fusion partner sequences.

The term "recombinant host cell" (or simply "host cell"), as used herein, is intended to refer to a cell that has been genetically engineered. A recombinant host cell includes a cell into which a recombinant vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. A recombinant host cell may be an isolated cell or cell line grown in culture or may be a cell which resides in a living tissue or organism. The term "host" refers to any organism or plant comprising one or more "host cells", or to the source of the "host cells".

Moreover, as used herein a "host cell which lac ks endogenous CMP-Sia" refers to a cell that does not endogeneously produce CMP-Sia, including cells which lack a CMP-Sia pathway. As used herein a "fungal host cell" refers to a fungal host cell that lacks CMP-Sia.

The term "peptide" as used herein refers to a short polypeptide, e.g., one that is typically less than about 50 amino acids long and more typically less than about 30 amino acids long. The term as used herein encompasses analogs and mimetics that mimic structural and thus biological function.

The term "polypeptide" encompasses both naturally-occurring and non-naturally-occurring proteins, and fragments, mutants, homologs, variants, derivatives and analogs thereof. A polypeptide may be monomeric or polymeric. Further, a polypeptide may comprise a number of different domains each of which has one or more distinct activities.

The term "isolated protein" or "isolated polypeptide" is a protein or polypeptide that by virtue of its origin or source of derivation (1) is not associated with naturally associated components that accompany it in its native state, (2) when it exists in a purity not found in nature, where purity can be adjudged with respect to the presence of other cellular material (e.g., is free of other proteins from the same species) (3) is expressed by a cell from a different species, or (4) does not occur in nature (e.g., it is a fragment of a polypeptide found in nature or it includes amino acid analogs or derivatives not found in nature or linkages other than standard peptide bonds). Thus, a polypeptide that is chemically synthesized or synthesized in a cellular system different from the cell from which it naturally originates will be "isolated" from its naturally associated components. A polypeptide or protein may also be rendered substantially free of naturally associated components by isolation, using protein purification techniques well known in the art. As thus defined, "isolated" does not necessarily require that the protein, polypeptide, peptide or oligopeptide so described has been physically removed from its native environment.

The term "polypeptide fragment" as used herein refers to a polypeptide that has an amino-terminal and/or carboxy-terminal deletion compared to a full-length polypeptide. In a preferred embodiment, the polypeptide fragment is a contiguous sequence in which the amino acid sequence of the fragment is identical to the corresponding positions in the naturally-occurring sequence. Fragments typically are at least 5, 6, 7, 8, 9 or 10 amino acids long, preferably at least 12,14, 16 or 18 amino acids long, more preferably at least 20 amino acids long, more preferably at least 25, 30, 35, 40 or 45, amino acids, even more preferably at least 50 or 60 amino acids long, and even more preferably at least 70 amino acids long.

A "recombinant protein", "recombinant glycoprotein" or "recombinant enzyme" refers to a protein, glycoprotein or enzyme (respectively) produced by genetic engineering. A recombinant protein, glycoprotein or enzyme includes a heterologous protein, glycoprotein or enzyme (respectively) expressed from a nucleic acid which has been introduced into a host cell.

A "modified derivative" or a "derivative" refers to polypeptides or fragments thereof that are substantially homologous in primary structural sequence but which include, e.g., *in vivo* or *in vitro* chemical and biochemical modifications or which incorporate amino acids that are not found in the native polypeptide. Such modifications include, for example, acetylation, carboxylation, phosphorylation, glycosylation, ubiquitination, labeling, e.g., with radionuclides, and various enzymatic modifications, as will be readily appreciated by those well skilled in the art. A variety of methods for labeling polypeptides and of substituents or labels useful for such purposes are well known in the art, and include radioactive isotopes such as ¹²⁵I, ³²P, ³⁵S, and ³H, ligands which bind to labeled antiligands (e.g., antibodies), fluorophores, chemiluminescent agents, enzymes, and antiligands which can serve as specific binding pair members for a labeled ligand. The choice of label depends on the sensitivity required, ease of conjugation with the primer, stability requirements, and available instrumentation. Methods for labeling polypeptides are well known in the art. See Ausubel et al., 1992, hereby incorporated by reference.

The term "fusion protein" refers to a polypeptide comprising a polypeptide or fragment coupled to heterologous amino acid sequences. Fusion proteins are useful because they can be constructed to contain two or more desired functional elements from two or more different proteins. A fusion protein comprises at least 10 contiguous amino acids from a polypeptide of interest, more preferably at least 20 or 30 amino acids, even more preferably at least 40, 50 or 60 amino acids, yet more preferably at least 75, 100 or 125 amino acids. Fusion proteins can be produced recombinantly by constructing a nucleic acid sequence which encodes the polypeptide or a fragment thereof in frame with a nucleic acid sequence encoding a different protein or peptide and then expressing the fusion protein. Alternatively, a fusion protein can be produced chemically by crosslinking the polypeptide or a fragment thereof to another protein.

The term "non-peptide analog" refers to a compound with properties that are analogous to those of a reference polypeptide. A non-peptide compound may also be termed a "peptide mimetic" or a "peptidomimetic". See, e.g., Jones, (1992) Amino Acid and Peptide Synthesis, Oxford University Press; Jung, (1997) Combinatorial Peptide and Nonpeptide Libraries: A Handbook, John Wiley; Bodanszky et al. (1993), Peptide Chemistry - A Practical Textbook Springer Verlag; "Synthetic Peptides: A Users Guide", G.A. Grant, Ed, W.H., Freeman and Co. (1992); Evans et al. J. Med. Chem. 30:1229 (1987); Fauchere, J. Adv. Drug Res. 15:29 (1986); Veber and Freidinger, TINS p.392 (1985); and references cited in each of the above. Such compounds are often developed with the aid of computerized molecular modeling. Peptide mimetics that are structurally similar to useful peptides of the invention may be used to produce an equivalent effect and are therefore envisioned to be part of the invention.

A "polypeptide mutant" or "mutein" or "variant" refers to a polypeptide whose sequence contains an insertion, duplication, deletion, rearrangement or substitution of one or more amino acids compared to the amino acid sequence of a native or wild type protein. A mutein may have one or more amino acid point substitutions, in which a single amino acid at a position has been changed to another amino acid, one or more insertions and/or deletions, in which one or more amino acids are inserted or deleted, respectively, in the sequence of the naturally-occurring protein, and/or truncations of the amino acid sequence at either or both the amino or carboxy termini. A mutein may have the same but preferably has a different biological activity compared to the naturally-occurring protein.

A mutein has at least 70% overall sequence homology to its wild-type counterpart. Even more preferred are muteins having 80%, 85% or 90% overall sequence homology to the wild-type protein. In an even more preferred embodiment, a mutein exhibits 95% sequence identity, even more preferably 97%, even more preferably 98% and even more preferably 99% overall sequence identity. Sequence homology may be measured by any common sequence analysis algorithm, such as Gap or Bestfit.

Preferred amino acid substitutions are those which: (1) reduce susceptibility to proteolysis, (2) reduce susceptibility to oxidati on, (3) alter binding affinity for forming protein complexes, (4) alter binding affinity or enzymatic activity, and (5) confer or modify other physicochemical or functional properties of such analogs.

As used herein, the twenty conventional amino acids and their abbreviations follow conventional usage. *See* Immunology - A Synthesis (2nd Edition, E.S. Golub and D.R. Gren, Eds., Sinauer Associates, S underland, Mass. (1991)). Stereoisomers (e.g., D-amino acids) of the twenty conventional amino acids, unnatural amino acids such as α-, α-disubstituted amino acids, N-alkyl amino acids, and other unconventional amino acids may also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, s-N-methylarginine, and other similar amino acids and imino acids (e.g., 4-hydroxyproline). In the polypeptide notation used herein, the left-hand direction is the amino terminal direction and the right hand direction is the carboxy-terminal direction, in accordance with standard usage and convention.

A protein has "homology" or is "homologous" to a second protein if the nucleic acid sequence that encodes the protein has a similar sequence to the nucleic acid sequence that encodes the second protein. Alternatively, a protein has homology to a second protein if the two proteins have "similar" amino acid sequences. (Thus, the term "homologous proteins" or "homologs" is defined to mean that the two proteins have similar amino acid sequences). In a preferred embodiment, a homologous protein is one that exhibits 50% sequence homology to the wild type protein, more preferred is 60% sequence homology. Even more preferred are homologous proteins that exhibit 80%, 85% or 90% sequence homology to the wild type protein. In a yet more preferred embodiment, a homologous protein exhibits 95%, 97%, 98% or 99% sequence identity. As used herein, homology between two regions of amino acid sequence (especially with respect to predicted structural similarities) is interpreted as implying similarity in function.

When "homologous" is used in reference to proteins or peptides, it is recognized that residue positions that are not identical often differ by conservative amino acid substitutions. A "conservative amino acid substitution" is one in which an amino acid residue is substituted by another amino acid residue having a side chain (R group) with similar chemical properties (e.g., charge or hydrophobicity). In general, a conservative amino acid substitution will not substantially change the functional properties of a protein. In cases where two or more amino acid sequences differ from each other by conservative substitutions, the percent sequence identity or degree of homology may be adjusted upwards to correct for the conservative nature of the substitution. Means for making this adjustment are well known to those of skill in the art (see, e.g., Pearson et al., 1994).

The following six groups each contain amino acids that are conservative substitutions for one another: 1) Serine (S), Threonine (T); 2) Aspartic Acid (D), Glutamic Acid (E); 3) Asparagine (N), Glutamine (Q); 4) Arginine (R), Lysine (K); 5) Isoleucine (I), Leucine (L), Methionine (M), Alanine (A), Valine (V), and 6) Phenylalanine (F), Tyrosine (Y), Tryptophan (W).

Sequence homology for polypeptides, which is also referred to as percent sequence identity, is typically measured using sequence analysis software. See, e.g., the Sequence Analysis Software Package of the Genetics Computer Group (GCG), University of Wisconsin Biotechnology Center, 910 University Avenue, Madison, Wisconsin 53705. Protein analysis software matches similar sequences using measure of homology assigned to various substitutions, deletions and other modifications, including conservative amino acid substitutions. For instance, GCG contains programs such as "Gap" and "Bestfit" which can be used with default parameters to determine sequence homology or sequence identity between closely related polypeptides, such as homologous polypeptides from different species of organisms or between a wild type protein and a mutein thereof. See, e.g., GCG Version 6.1.

A preferred algorithm when comparing a inhibitory molecule sequence to a database containing a large number of sequences from different organisms is the computer program BLAST (Altschul, S.F. et al. (1990) J. Mol. Biol. 215:403-410; Gish and States (1993) Nature Genet. 3:266-272; Madden, T.L. et al. (1996) Meth. Enzymol. 266:131-141; Altschul, S.F. et al. (1997) Nucleic Acids Res.25:3389-3402; Zhang, J. and Madden, T.L. (1997) Genome Res. 7:649-656, especially blastp or tblastn (Altschul et al., 1997)). Preferred parameters for BLASTp are: Expectation value: 10 (default); Filter: seg (default); Cost to open a gap: 11 (default); Cost to extend a gap: 1 (default; Max. alignments: 100 (default); Word size: 11 (default); No. of descriptions: 100 (default); Penalty Matrix: BLOWSUM62.

The length of polypeptide sequences compared for homology will generally be at least about 16 amino acid residues, usually at least about 20 residues, more usually at least about 24 residues, typically at least about 28 residues, and preferably more than about 35 residues. When searching a database containing sequences from a large number of different organisms, it is preferable to compare amino acid sequences. Database searching using amino acid sequences can be measured by algorithms other than blastp known in the art. For instance, polypeptide sequences can be compared using FASTA, a program in GCG Version 6.1. FASTA provides alignments and percent sequence identity of the regions of the best overlap between the query and search sequences (Pearson, 1990, herein incorporated by reference). For example, percent sequence identity between amino acid sequences can be determined using FASTA with its default parameters (a word size of 2 and the PAM250 scoring matrix), as provided in GCG Version 6.1.

"Specific binding" refers to the ability of two molecules to bind to each other in preference to binding to other molecules in the environment Typically, "specific binding" discriminates over adventitious binding in a reaction by at least two-fold, more typically by at least 10-fold, often at least 100-fold. Typically, the affinity or avidity of a specific binding reaction is at least about 10⁻⁷ M (e.g., at least about 10⁻⁸ M or 10⁻⁹ M).

The term "region" as used herein refers to a physically contiguous portion of the primary structure of a biomolecule. In the case of proteins, a region is defined by a contiguous portion af the amino acid sequence of that protein.

The term "domain" as used herein refers to a structure of a biomolecule that contributes to a known or suspected function of the biomolecule. Domains may be co-extensive with regions or portions thereof; domains may also include distinct, non-contiguous regions of a biomolecule. Examples of protein domains include, but are not limited to, an Ig domain, an extracellular domain, a transmembrane domain, and a cytoplasmic domain.

As used herein, the term "molecule" means any compound, including, but not limited to, a small molecule, peptide, protein, sugar, nucleotide, nucleic acid, lipid, etc., and such a compound can be natural or synthetic.

As used herein, a "CMP-Sialic acid biosynthetic pathway" or a "CMP-Sia biosynthetic pathway" refers to one or more glycosylation enzymes which results in the formation of CMP-Sia in a host

As used herein, a "CMP-Sia pool" refers to a detectable level of cellular CMP-Sia.

As used herein, the term "N-glycan" refers to an N-linked oligosaccharide, e.g., one that is attached by an asparagine-N-acetylglucosamine linkage to an asparagine residue of a polypeptide. N-glycans have a common pentasaccharide core of Man₃GlcNAc₂ ("Man" refers to mannose; "Glc" refers to glucose; and "NAc" refers to N-acetyl; GlcNAc refers to N-acetylglucosamine). The term "trimannose core" used with respect to the N-glycan also refers to the structure Man₃GlcNAc₂ ("Man3"). N-glycans differ with respect to the number of branches (antennae) comprising peripheral sugars (e.g., GlcNAc, galactose and sialic acid) that are added to the Man₃ core structure. N-glycans are classified according to their branched constituents (e.g., high mannose, complex or hybrid).

A "high mannose" type N-glycan has five or more mannose residues. A "complex" type N-glycan typically has at least one GlcNAc attached to the 1,3 mannose arm and at least one GlcNAc attached to the 1,6 mannose arm of the trimannose core. Complex N-glycans may also have galactose ("Gal") residues that are optionally modified with sialic acid or derivatives ("NeuAc", where "Neu" refers to neuraminic acid and "Ac" refers to acetyl). A complex N-glycan typically has at least one branch that terminates in an oligosaccharide such as, for example: NeuAc-; NeuAcα2-6GalNAcα1-; NeuAcα2-3Gal*β*1-3GalNAcα1-; NeuAcα2-3/6Gal*β*1-4GlcNAc*β*1-; GlcNAcα1-4Gal*β*1-(mucins only); Fucα1-2Gal*β*1-(blood group H). Sulfate esters can occur on galactose, GalNAc, and GlcNAc residues, and phosphate esters can occur on mannose residues. NeuAc (Neu: neuraminic acid; Ac:acetyl) can be O-acetylated or replaced by NeuGl (N-glycolylneuraminic acid). Complex N-glycans may also have intrachain substitutions comprising "bisecting" GlcNAc and core fucose ("Fuc"). A "hybrid" N-glycan has at least one GlcNAc on the terminal of the 1,3 mannose arm of the trimannose core and zero or more mannoses on the 1,6 mannose arm of the trimannose core.

The substrate UDP-GlcNAc is the abbreviation for UDP-*N-*acetylglucosamine. The intermediate ManNAc is the abbreviation for *N-*acetylmannosamine. The intermediate ManNAc-6-P is the abbreviation for *N-*acetylmannosamine-6-phosphate. The intermediate Sia-9-P is the abbreviation for sialate-9-phosphate. The intermediate Cytidine monophosphate-sialic acid is abbreviated as "CMP-Sia." Sialic acid is abbreviated as "Sia," "Neu5Ac," "NeuAc"or "NANA" herein.

As used herein, the term "sialic acid" refers to a group of molecules where the common molecule includes N-acetyl-5-neuraminic acid (Neu5Ac) having the basic 9-carbon neuraminic acid core modified at the 5-carbon position with an attached acetyl group. Common derivatives of Neu5Ac at the 5-carbon position include: 2-keto-3-deoxy-d-glycero-d-galactonononic acid (KDN) which possesses a hydroxyl group in place of the acetyl group; de-N-acetylation of the 5-N-acetyl group produces neuraminic (Neu); hydroxylation of the 5-N-acetyl group produces N-glycolylneuraminic acid (Neu5Gc). The hydroxyl groups at positions 4-, 7-, 8-and 9- of these four molecules (Neu5Ac, KDN, Neu and Neu5Gc) can be further substituted with O-acetyl, O-methyl, O-sulfate and phosphate groups to enlarge this group of compounds. Furthermore, unsaturated and dehydro forms of sialic acids are known to exist.

The gene encoding for the UDP-GlcNAc epimerase is abbreviated as *"NeuC."* The gene encoding for the sialate synthase is abbreviated as "*NeuB.*" The gene encoding for the CMP-Sialate synthase is abbreviated as "*NeuA.*"

Sialate aldolase is also commonly referred to as sialate lyase and sialate pyruvate-lyase. More specifically in *E*. *coli*, sialate aldolase is referred to as *NanA.*

The term "enzyme," when used herein in connection with altering host cell glycosylation, refers to a molecule having at least one enzymatic activity, and includes full-length enzymes, catalytically active fragments, chimerics, complexes, and the like.

A "catalytically active fragment" of an enzyme refers to a polypeptide having a detectable level of functional (enzymatic) activity.

As used herein, the term "secretion pathway" refers to the assembly line of various glycosylation enzymes to which a lipid-linked oligosaccharide precursor and an N-glycan substrate are sequentially exposed, following the molecular flow of a nascent polypeptide chain from the cytoplasm to the endoplasmic reticulum (ER) and the compartments of the Golgi apparatus. Enzymes are said to be localized along this pathway. An enzyme X that acts on a lipid-linked glycan or an N-glycan before enzyme Y is said to be or to act "upstream" to enzyme Y; similarly, enzyme Y is or acts "downstream" from enzyme X.

The term "polynucleotide" or "nucleic acid molecule" refers to a polymeric form of nucleotides of at least 10 bases in length. The term includes DNA molecules (e.g., cDNA or genomic or synthetic DNA) and RNA molecules (e.g., mRNA or synthetic RNA), as well as analogs of DNA or RNA containing non-natural nucleotide analogs, non-native internucleoside bonds, or both. The nucleic acid can be in any topological conformation. For instance, the nucleic acid can be single-stranded, double-stranded, triple-stranded, quadruplexed, partially double-stranded, branched, hairpinned, circular, or in a padlocked conformation. The term includes single and double stranded forms of DNA. A nucleic acid molecule disclosed herein may include both sense and antisense strands of RNA, cDNA, genomic DNA, and synthetic forms and mixed polymers of the above. They may be modified chemically or biochemically or may contain non-natural or derivatized nucleotide bases, as will be readily appreciated by those of skill in the art Such modifications include, for example, labels, methylation, substitution of one or more of the naturally occurring nucleotides with an analog, internucleotide modifications such as uncharged linkages (e.g., methyl phosphonates, phosphotriesters, phosphoramidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.) Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

Unless otherwise defined, all technical and scientific terms used herein have the same meaning as commonly understood by one of ordinary skill in the art to which this invention pertains.

Throughout this specification and claims, the word "comprise" or variations such as "comprises" or "comprising", will be understood to imply the inclusion of a stated integer or group of integers but not the exclusion of any other integer or group of integers.

### Methods For Producing CMP-Sia For The Generation Of Recombinant N-Glycans In Fungal Cells

The present invention provides methods for production of a functional CMP-Sia biosynthetic pathway in a host cell which lacks endogenous CMP-Sia, such as a fungal cell. The present invention also provides a method for creating a host which has been modified to express a CMP-Sia pathway. The invention further provides a method for creating a host cell which comprises a cellular pool of CMP-Sia.

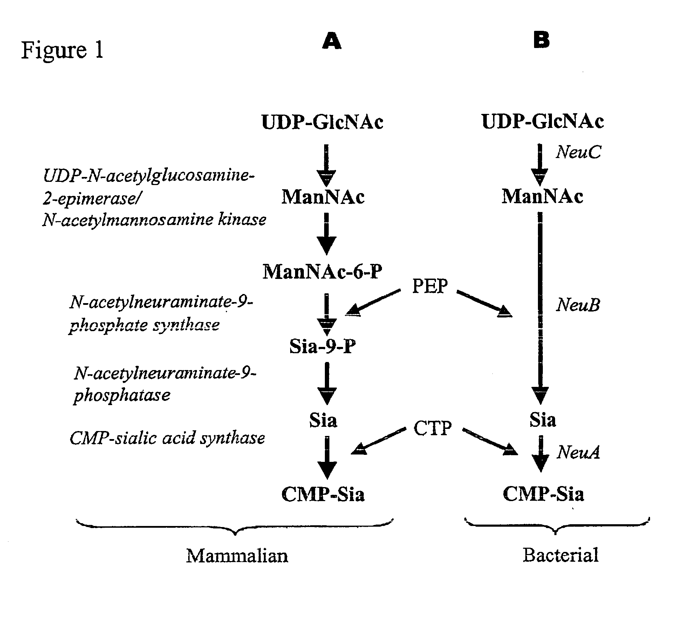

The methods involve the cloning and expression of several genes encoding enzymes of the CMP-Sia biosynthetic pathway resulting in a cellular pool of CMP-Sia which can be utilized in the production of sialylated glycans on proteins of interest. In general, the addition of sialic acids to glycans requires the presence of the sialyltransferase, a glycan acceptor (e.g., Gal₂GlcNAc₂Man₃GlcNAc₂) and the sialyl donor molecule, CMP-Sia. The synthesis of the CMP-Sia donor molecule in higher organisms (e.g., mammals) is a four enzyme, multiple reaction process starting with the substrate UDP-GlcNAc and resulting in CMP-Sia (**Figure 1A**). The process initiates in the cytoplasm producing sialic acid which is then translocated into the nucleus where Sia is converted to CMP-Sia. Subsequently, CMP-Sia exits the nucleus into the cytoplasm and is then transported into the Golgi where sialyltransferases catalyze the transfer of sialic acid onto the acceptor glycan. In contrast, the bacterial pathway for synthesizing CMP-Sia from UDP-GlcNAc involves only three enzymes and two intermediates (**Figure 1B**), with all reactions occurring in the cytoplasm.

Accordingly, the methods of the invention involve generating a pool of CMP-Sia in a non-human host cell which lacks endogenous CMP-Sia by introducing a functional CMP-Sia biosynthetic pathway. With readily available DNA sequence information from genetic databases (e.g., GenBank, Swissprot), enzymes and/or activities involved in the CMP-Sia pathways (**Example 1**) are cloned. Using standard techniques known to those skilled in the art, nucleic acid molecules encoding enzymes (or catalytically active fragments thereof) involved in the biosynthesis of CMP-Sia are inserted into appropriate expression vectors under the transcriptional control of promoters and/or other expression control sequences capable of driving transcription in a selected host cell of the invention (e.g., a yeast host cell). The functional expression of such enzymes in the selected host cells of the invention can be detected. In one embodiment, the functional expression of such enzymes in the selected host cells can be detected by measuring the intermediate formed by the enzyme. The methods diæloeedherein are not limited to the use of the specific enzyme sources disclosed herein.

### Engineering A Mammalian CMP-Sialic Acid Biosynthetic Pathway In Fungi

In one aspect of the invention, a method for synthesizing a mammalian CMP-sialic acid pathway in a host cell which lacks endogenous CMP-Sia is provided. In mammals and higher eukaryotes, synthesis of CMP-sialic acid is initiated in the cytoplasm where the enzyme activities (UDP-N-acetyl-glucosamine-2-epimerase/N-acetylmannosamine kinase, N-acetylneuraminate-9-phosphate synthase, N-acetylneuraminate-9-phosphatase) convert UDP-GlcNAc to sialic acid (**Figure 1A**). The sialic acid then enters the nucleus where it is converted to CMP-sialic acid by CMP-sialic acid synthase.

In one embodiment disclosed herein, the method involves cloning several genes encoding enzymes in the CMP-Sia biosynthetic pathway, including UDP-N-acetylglucosamine-2-epimerase/N-acetylmannosamine kinase, N-acetylneuraminate-9-ghosphate synthase, N-acetylneuraminate-9-phosphatase, and CMP-sialic acid synthase, in a host cell which lacks endogenous CMP-Sia, such as a fungal host cell. The genes are expressed to generate each enzyme, producing intermediates that are used for subsequent enzymatic reactions. **Examples 5-8** describe methods for the introduction of these enzymes into a fungal host (e.g., *P*. *pastoris*) using a selection marker. Alternatively, the enzymes are expresses together to produce or increase downstream intermediates whereby subsequent enzymes are able to act upon them.

The first enzyme in the pathway is a bi-functional enzyme that is both an UDP-GlcNAc epimerase and an N-acetylmannosamine kinase, converting UDP-GlcNac through N-acetylmannosamine (ManNAc) to N-acetylmannosamine-6-phosphate (ManNAc-6-P) (Hinderlich, S., Stasche, R., et al. 1997). This enzyme was originally cloned from a rat liver cDNA library (Stasche, R., Hinderlich, S., et al. 1997). In a preferred embodiment, a gene encoding the functional UDP-N-acetylglucosamine-2-epimerase enzyme, including homologs, variants and derivatives thereof, is cloned and expressed in a non-human host cell which lacks endogenous CMP-Sia, such as a fungal host cell. In another preferred embodiment, a gene encoding the functional N-acetylmannosamine kinase enzyme, including homologs, variants and derivatives thereof, is cloned and expressed in a host cell, such as a fungal host cell. In a more preferred embodiment, a gene encoding the bifunctional UDP-N-acetylglucosamine-2-epimerase/N-acetylmannosamine kinase enzyme, including homologs, variants and derivatives thereof, is cloned and expressed in a non-human host cell which lacks endogenous CMP-Sia, such as a fungal host cell (e.g., *P. pastoris*)*.* The functional expression of these genes can be detected using a functional assay. In one embodiment, the functional expression of such genes can be detected by detecting the formation of ManNAc and ManNAc-6-P intermediates.

The second enzyme in the pathway, N-acetylneuraminic acid phosphate synthase, was cloned from human liver based on its homology to the *E*. *coli* sialic acid synthase gene, *NeuB* (Lawrence, S.M., Huddleston, K.A., et al. 2000). This enzyme catalyzes the conversion of ManNAc-6-P to sialate 9-phosphate (also referred to as Sia-9P, N-acetylneuraminate 9-phosphate, or Neu5Ac-9P). Accordingly, in a preferred embodiment, a gene encoding the functional N-acetylneuraminate 9-phosphate synthase enzyme, including homologs, variants and derivatives thereof, is cloned and expressed in a non-human host cell which lacks endogenous CMP-Sia, such as a fungal host cell. The functional expression N-acetylneuraminic acid phosphate synthase in the host can be detected using a functional assay. In one embodiment, the functional expression of N-acetylneuraminic acid phosphate synthase can be detected by detecting the formation of Sia-9P.

The third enzyme in the pathway, N-acetylneuraminate 9-phosphatase (Sia-9-phosphatase), has yet to be cloned but is involved in the conversion of Sia-9-P 9-P to sialic acid. Although the activity of this enzyme has been detected in mammalian cells, no such activity has been identified in fungal cells. Therefore, the lack of Sia-9-phosphatase would cause a break in the pathway. Accordingly, in a preferred embodiment, the method disclosed herein involves isolating and cloning a Sia-9-phosphatase gene into a non-human host cell, such as a fungal host cell. Such hosts include yeast, fungal, insect and bacterial cells. In a more preferred embodiment, the Sia-9-phosphatase gene, including homologs, variants and derivatives thereof is expressed in a non-human host cell which lacks endogenous CMP-Sia, such as a fungal host. The functional expression of Sia-9-phosphatase in the host can be detected using a functional assay. In one embodiment, the functional expression of Sia-9-phosphatase can be detected by detecting the formation of sialic acid.

The next enzyme in the mammalian pathway, CMP-Sia synthase, was originally cloned from the murine pituitary gland by functional complementation of a cell line deficient in this enzyme (Munster, A.K., Eckhardt, M., et al. 1998). This enzyme converts sialic acid to CMP-Sia, which is the donor substrate in a sialyltransferase reaction in the Golgi. Accordingly, in an even more preferred embodiment, a gene encoding the functional CMP-Sia synthase enzyme, including homologs, variants and derivatives thereof, is cloned and expressed in a non-human host cell which lacks endogenous CMP-Sia, such as a fungal host cell. The functional expression of CMP-Sia synthase synthase in the host can be detected using a functional assay. In one embodiment, the functional expression of CMP-Sia synthase can be detected by detecting the formation of CMP-Sia.

The method disclosed herein further involves the production of the intermediates produced in a non-human host as a result of expressing the above enzymes in the CMP-Sia pathway. Preferably, the intermediates produced include one or more of the following: UDP-GlcNAc, ManNAc, ManNAc-6-P, Sia-9-P, Sia and CMP-Sia. Additionally, each intermediate produced by the enzymes is preferably detected. For example, to detect the presence or absence of an intermediate, an assay as described in **Example 10** is used. Accordingly, the method also involves assays to detect the N-glycan intermediates produced in a non-human host cell which lacks endogenous CMP-Sia, such as a fungal host cell.

A skilled artisan recognizes that the mere availability of one or more enzymes in the CMP-sialic acid biosynthetic pathway does not suggest that such enzymes can be functionally expressed in a host cell which lacks endogenous CMP-Sia, such as a fungal host cell. To date, the ability of such host cell to express these mammalian enzymes to create a functional *de novo* CMP-Sia biosynthetic pathway has not been described. The present disclosure provides for the first time the functional expression of at least one mammalian enzyme involved in CMP-Sia biosynthesis in a fungal host: the mouse CMP-Sia synthase (**Example 8**), suggesting that production of CMP-Sia via the mammalian pathway (in whole or in part) is possible in a fungal host and in other non-human hosts which lack endogenous CMP-Sia.

The present disclosure is not limited to the use of the specific enzymes, genes, plasmids and constructs disclosed herein. A person of skill could use any homologs, variants and derivatives of the genes involved in the synthesis of CMP-Sia.

To produce sialylated, recombinant glycoproteins in a non-human host cell which lacks endogenous CMP-Sia (e.g., a fungal host such as *P. pastoris*), the above mentioned mammalian enzymes can be expressed using a combinatorial DNA library as disclosed in WO 02/00879, generating a pool of CMP-Sia, which is transferred onto galactosylated N-glycans in the presence of a sialyltransferase. Accordingly, the present disclosure provides a method for engineering a CMP-Sia biosynthetic pathway into a fungal host by expressing each of the enzymes such that they function, preferably so that they function optimally, in the fungal host. Mammalian, bacterial or hybrid engineered CMP-Sia biosynthetic pathways are provided.

### Engineering A Bacterial CMP-Sialic Acid Biosynthetic Pathway In Fungi

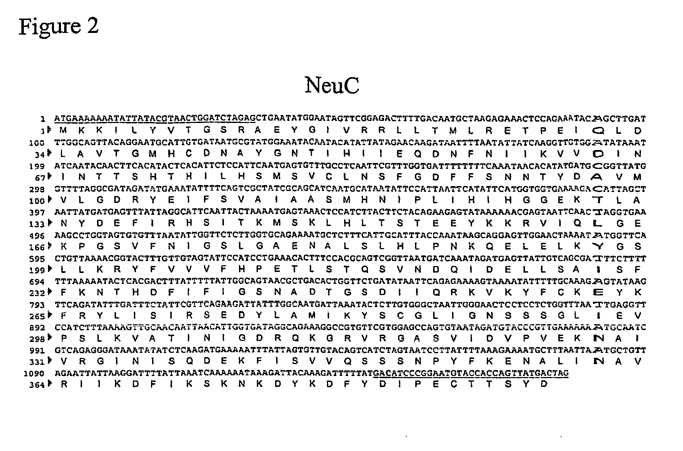

The metabolic intermediate UDP-GlcNAc is common to eukaryotes and prokaryotes, providing an endogenous substrate from which to initiate the synthesis of CMP-Sia (**Figure 1**). Based on the presence of this common intermediate, the CMP-Sia biosynthetic pathway can be engineered into non-human host cells which lack endogenous CMP-Sia by integrating the genes encoding the bacterial UDP-GlcNAc epimerase, sialate synthase and CMP-Sia synthase. Accordingly, another aspect of the present invention involves engineering the bacterial CMP-Sia biosynthetic pathway into host cells which lack an endogenous CMP-Sia pathway. The expression of bacterial *Neu* genes in cells which lack an endogenous CMP-Sia biosynthetic pathway enables the generation of a cellular CMP-Sia pool, which can subsequently facilitate the production of recombinant N-glycans having detectable level of sialylation on a protein of interest, such as recombinantly expressed glycoproteins. The bacterial enzymes involved in the synthesis of CMP-Sia include UDP-GlcNAc epimerase (*NeuC*), sialate synthase (*NeuB*) and CMP-Sia synthase (*NeuA*). In one embodiment, the *NeuC, NeuB,* and *NeuA* genes which encode these functional enzymes, respectively, including homologs, variants and derivatives thereof, are cloned and expressed in non-human host cells which lack an endogenous CMP-Sia pathway, such as a fungal host. The sequences of *NeuC, NeuB* and *NeuA* genes are shown in **Figures 2-4**, respectively. The expression of these genes generates the intermediate molecules in the biosynthetic pathway of CMP-sialic acid (**Figure 1B**).

In addition to these three enzymes, the method for synthesizing the bacterial CMP-Sia biosynthetic pathway from UDP-GlcNAc involves generating two intermediates: ManNAc and Sia (**Figure 1B**). The conversion of UDP-GlcNAc to ManNAc is facilitated by the *NeuC* gene. The conversion of ManNAc to Sia is facilitated by the *NeuB* gene and the conversion of substrates Sia to CMP-Sia is facilitated by the *NeuA* gene. These three enzymes (or homologs thereof) have thus far been found together in pathogenic bacteria -- i.e., not one of the genes has not been found without the other two. In comparison to the mammalian pathway, the introduction of the bacterial pathway into a host, such as a fungal host, requires the manipulation of fewer genes.

The *E*. *coli* UDP-GlcNAc epimerase, encoded by the *E*. *coli NeuC* gene, is the first enzyme involved in the bacterial synthesis of polysialic acid (Ringenberg, M., Lichtensteiger, C., et al. 2001). The *NeuC* gene (Genbank: M84026.1; SEQ ID NO:13) encoding this enzyme was isolated from the pathogenic *E*. *coli* Kl strain and encodes a protein of 391 amino acids (SEQ ID NO:14) (**Figure 2**) (Zapata, G., Crowley, J.M., et al. 1992). The encoded UDP-GlcNAc epimerase catalyzes the conversion of UDP-GlcNAc to ManNAc. Homologs of this enzyme have been identified in several pathogenic bacteria, including *Streptococcus agalactiae, Synechococcus* sp. WH 8102, *Clostridium thermocellum, Vibrio vulnificus, Legionella pnuemophila,* and *Campylobacter jejuni.* In one embodiment, a gene encoding the functional *E*. *coli* UDP-GlcNAc epimerase enzyme (*NeuC*), including homologs, variants and derivatives thereof, is cloned and expressed in a non-human host cell, such as a fungal host. The functional expression of *NeuC* in the host can be detected using a functional assay. In one embodiment, the functional expression *NeuC* can be detected by detecting the formation of ManNAc.

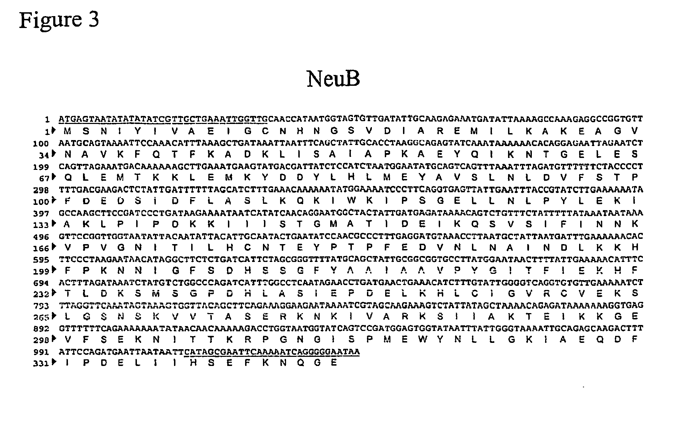

The second enzyme in the bacterial pathway is sialate synthase which directly converts ManNAc to Sia, bypassing several enzymes and intermediates present in the mammalian pathway. This enzyme of 346 amino acids (SEQ ID NO:16), is encoded by the *E*. *coli NeuB* gene (Genbank: U05248.1; SEQ ID NO:15) (**Figure 3**) (Annunziato, P.W., Wright, L.F., et al. 1995). In another embodiment, a gene encoding a functional *E*. *coli* sialate synthase enzyme (*NeuB*), including homologs, variants and derivatives thereof, is cloned and expressed in a non-human host cell, such as a fungal host cell. The functional expression of *NeuB* in the host can be detected using a functional assay. In one embodiment, the functional expression *NeuB* can be detected by detecting the formation of Sia.

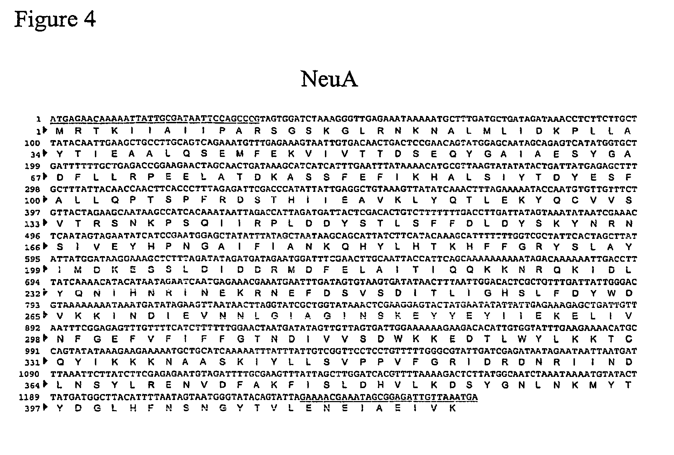

The third enzyme in this bacterial pathway is CMP-Sia synthase, consisting of 419 amino acids (SEQ ID NO:18) and encoded by the *E*. *coli NeuA* gene (Genbank: J05023; SEQ ID NO:17) (Figure 4). CMP-Sia synthase converts Sia to CMP-Sia (Zapata, G., Vann, W.F., et al. 1989). The *NeuA* gene is found in the same organisms as the *NeuC* and *NeuB* genes. Accordingly, in yet another embodiment, a gene (*NeuA*) encoding a functional *E*. *coli* CMP-Sia synthase enzyme, including homologs, variants and derivatives thereof, is cloned and expressed in a non-human host cell, such as a fungal host cell. In one embodiment, the functional expression *NeuA* can be detected by detecting the formation of CMP-Sia.

In yet another embodiment, the gene encoding a functional bacterial CMP-Sia synthase (e.g. *NeuA*) encodes a fusion protein comprising a: catalytic domain having the activity of a bacterial CMP-Sia synthase and a cellular targeting signal peptide (not normally associated with the catalytic domain) selected to target the enzyme to the nucleus of the host cell. In one embodiment, said cellular targeting signal peptide comprises a domain of the SV40 capside polypeptide VP1. In another embodiment, the signal peptide comprises one or more endogenous signaling motifs from a mammalian CMP-Sia synthase that ensure correct localization of the enzyme to the nucleus. The methods of making said fusion protein are well known in the art.

After PCR amplification of the *E*. *coli NeuA, NeuB* and *NeuC* genes, the amplified fragments were ligated into a selectable yeast integration vector under the control of a promoter (**Example 2**). After transforming a host strain (e.g.,
*P. pastoris*)*,* with each vector carrying the *Neu* gene fragments, colonies were screened by applying positive selection. These transformants were grown in YPD media. An assay for *Neu* gene enzymatic activity is carried out after each transformation. The ability of a non-human host which lacks endogenous sialylation to express the bacterial enzymes involved in creating a *de novo* CMP-Sia biosynthetic pathway is provided for the first time herein.

### Engineering A Hybrid Mammalian/Bacterial CMP-Sialic Acid Biosynthetic Pathway In Fungi

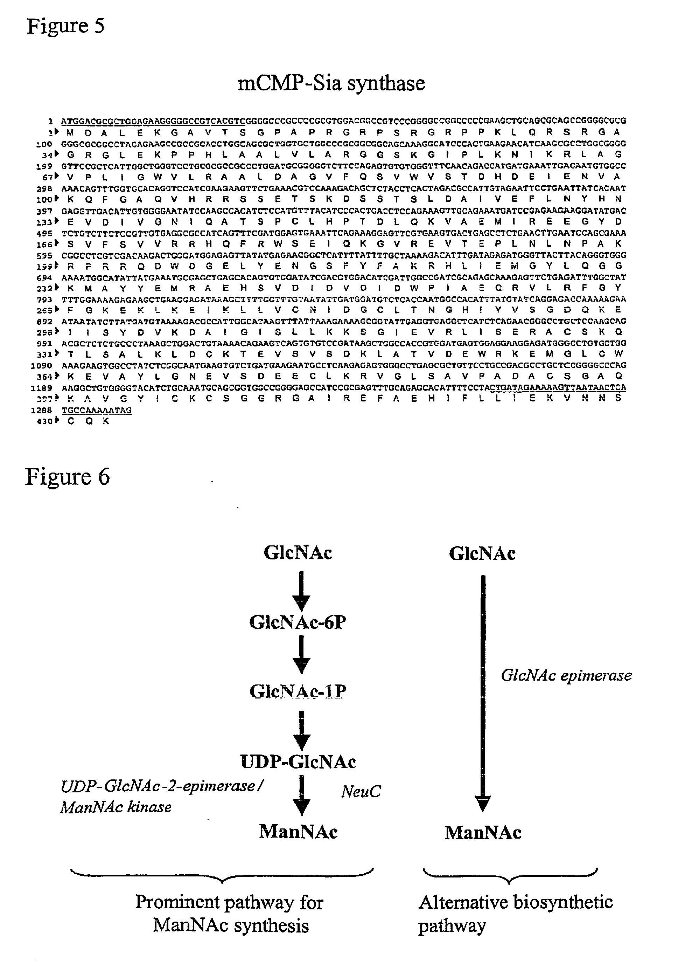

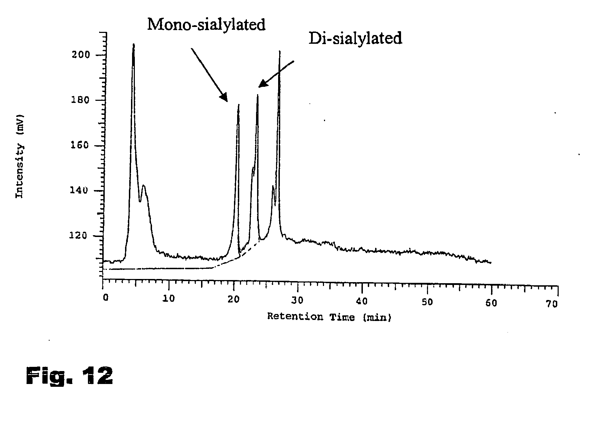

Both mammalian and bacterial CMP-Sia biosynthetic pathways require that both CTP and sialic acid be available to the CMP-Sia synthase. Although similar in enzymatic function to the corresponding bacterial enzyme, the mammalian CMP-Sia synthase may include one or more endogenous signaling motifs that ensure correct localization to the nucleus. Because eukaryotes have a nucleus-localized pool of CTP and the prokaryotic CMP-Sia synthase may not localize to this compartment, a hybrid CMP-Sia biosynthetic pathway combining both mammalian and bacterial enzymes is a preferred method for the production of sialic acid and its intermediates in a non-human host cell, such as a fungal host cell. To this end, a pathway can be engineered into the host cell which involves the integration of both *NeuC* and *NeuB* as well as a mammalian CMP-Sia synthase. The CMP-Sia synthase enzyme may be selected from several mammalian homologs that have been cloned and characterized (Genbank: AJ006215; SEQ ID NO:19) (Munster, A.K., Eckhardt, M., et al. 1998) (see e.g., the murine CMP-Sia synthase) (**Figure 5**). Preferably, the host cell is transformed with UDP-GlcNAc epimerase (*E*. *coli NeuC*) and sialate synthase (*E*. *coli NeuB*) in combination with the mouse CMP-Sia synthase. The host engineered with this hybrid CMP-Sia biosynthetic pathway produces a cellular pool of the donor molecule CMP-Sia (**Figure 12**). In a more preferred embodiment, the combination of the enzymes expressed in the host enhances production of the donor molecule CMP-Sia.

### Engineering Enzymes Involved In Alternative Routes For Enhancing The Production Of CMP-Sialic Acid Pathway Intermediates In Fungi

In yet another aspect of the disclosure enzymes involved in alternate pathways of CMP-sialic acid biosynthesis are engineered into non-human host cells, such as fungal host cells. For example, it is contemplated that when an intermediate becomes limiting during one of the methods outlined above, the introduction of an enzyme that uses an alternate mechanism to produce that intermediate will serve as a sufficient substitute in the production of CMP-sialic acid, or any intermediate along this pathway. Embodiments are described herein for the production of the intermediates ManNAc and Sia, though this approach may be extended to produce other intermediates. Furthermore, any of these enzymes can be incorporated into either the mammalian, bacterial or hybrid pathways, either in the absence of the enzymes mentioned previously (i.e., enzymes producing the same intermediate) or in the presence of enzymes mentioned previously, i.e., to enhance overall production.

In the above mentioned embodiments, ManNAc is produced from UDP-GlcNAc by either the mammalian enzyme UDP-GlcNAc-2-epimerase/ManNAc kinase or by the bacterial enzyme *NeuC.* The substrate for this reaction, UDP-GlcNAc, is predicted to be present in sufficient quantities in cells for the synthesis of CMP-Sia due to its requirement in producing several classes of molecules, including endogenous N-glycans. However, if ManNAc does become limiting - potentially due to the increased demand for ManNAc from the sialic acid biosynthetic pathway - then the cellular supply of ManNAc may be increased by introducing a GlcNAc epitnerase which reacts with the substrate GlcNAc to produce ManNAc.

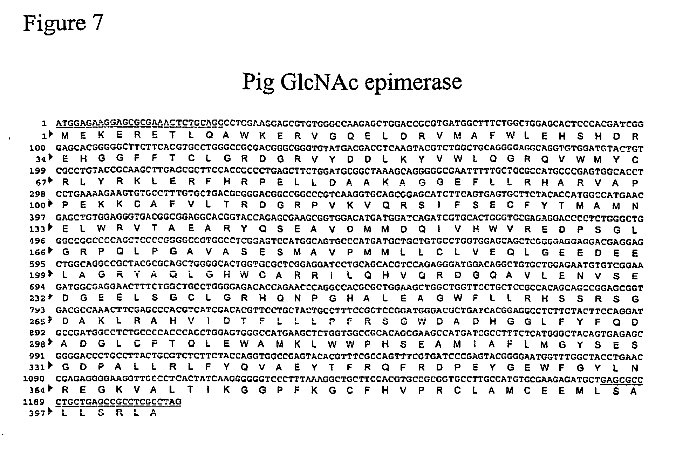

Accordingly, in one embodiment, a gene encoding a functional GlcNAc epimerase enzyme, including homologs, variants and derivatives thereof, is cloned and expressed in a host cell, such as a fungal host cell. Using GlcNAc epimerase to directly convert GlcNAc to ManNAc is a shorter, more efficient approach compared with the two-step process involving the synthesis of UDP-GlcNAc (**Figure 6**). The GlcNAc epimerase is readily available and, to date, the only confirmed GlcNAc epimerase to have been cloned is from the pig kidney (Maru, I., Ohta, Y., et al. 1996) (**Example 3**). The gene (Genbank: D83766; SEQ ID NO: 21) isolated from pig kidney encodes a protein of 402 amino acids (SEQ ID NO:22) (**Figure 7**). When this enzyme was cloned, it was found to be identical to the pig renin-binding protein cloned previously (Inoue, H., Fukui, K., et al. 1990). Although this is the only protein with confirmed GlcNAc epimerase activity, several other renin-binding proteins have been isolated from other organisms, including humans, mouse, rat and bacteria, among others. All are shown to have significant homology. For example, the human GlcNAc epimerase homolog (Genbank: D10232.1) has 87% identity and 92% similarity to the pig GlcNAc epimerase protein. Although these homologs are very similar in sequence, the pig protein is the only one having demonstrable epimerase activity to date. The methods of the invention could be performed using any gene encoding a functional GlcNAc epimerase activity. Based on the presence of the activity of GlcNAc epimerase, the cloning and expression of this gene in a non-human host cell, such as a fungal host cell, is predicted to enhance the cellular levels of ManNAc, thereby, providing sufficient substrate for the enzymes that utilize ManNAc in the CMP-sialic acid biosynthetic pathway.

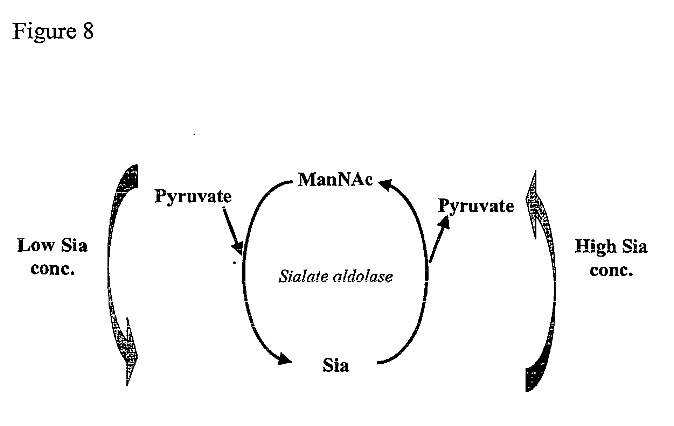

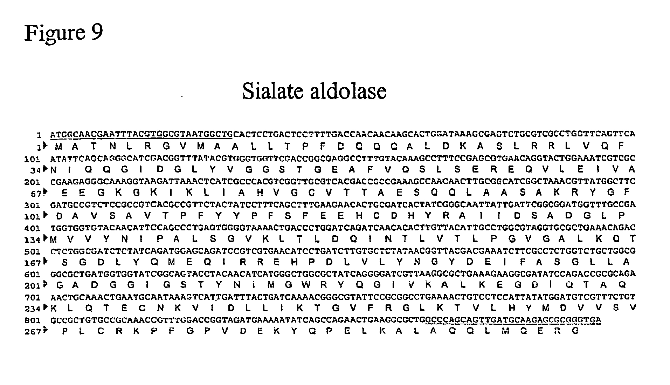

In another embodiment, sialate aldolase is used to increase cellular levels of sialic acid, as illustrated in **Figure 8**. This enzyme (also known as sialate lyase and sialate pyruvate-lyase) directly catalyzes the reversible reaction ofManNAc to sialic acid. In the presence of low concentrations of Sia, this enzyme catalyzes the condensation of ManNAc and pyruvate to produce Sia. Conversely, when Sia concentrations are high, the enzyme causes the reverse reaction to proceed, producing ManNAc and pyruvate (Vimr, E.R. and Troy, F.A. 1985). In the above embodiments, the presence of CMP-Sia synthase converts substantially all Sia to CMP-Sia, thus shifting the equilibrium of the aldolase to the condensation of ManNAc and pyruvate to produce Sia. Preferably, the sialate aldolase used in this embodiment is expressed from the *E*. *coli NanA* gene, but the invention is not limited to this enzyme source. The gene (Genbank: X03345; SEQ ID NO:23) for this enzyme encodes a 297 amino acid protein (SEQ ID NO:24) (**Figure 9**) (Ohta, Y., Watanabe, K. et al. 1985). Close homologs to this enzyme are found in many pathogenic bacteria, including, *Salmonella typhimurium, Staphylococcus aureus, Clostridium perfringens, Haemophilus influenzae* among others. In addition, homologs are also present in mammals, including mice and humans. Cloning a gene encoding a sialate aldolase activity and expressing it in a fungal host cellenhances the cellular levels of Sia, thereby providing sufficient substrate for the enzymes that utilize Sia in the CMP-sialic acid biosynthetic pathway (**Example 4**).

### Regulation Of CMP-Sialic Acid Synthesis: Feedback Inhibition And Inducible Promoters

In mammalian cells, the production of CMP-sialic acid is highly regulated. CMP-sialic acid acts as a feedback inhibitor, acting on UDP-GlcNAc epimerase/ManNAc kinase to prevent further production of CMP-Sia (Hinderlich, S., Stasche, R., et al. 1997) (Keppler, O.T., Hinderlich, S. et al., 1999). In contrast, the bacterial CMP-Sia biosynthetic pathway (**Figure 1B**) does not appear to have a feedback inhibitory control mechanism that would limit the production of CMP-Sia (Ringenberg, M., Lichtensteiger, C. et al. 2001). However, incorporation of the *E. coli* sialate aldolase into one of the pathways mentioned above could cause a shift in the direction of the reaction that it catalyzes, depending on the balance of the equilibrium, thus potentially causing hydrolysis of Sia back to ManNAc. Accordingly, the methods involving sialate aldolase as outlined above will prevent this reverse reaction from occurring, given the presence of CMP-sialate synthase which rapidly converts Sia to CMP-Sia.

The embodiments described thus far have detailed the constitutive over-expression of the enzymes in a particular biosynthetic pathway of CMP-Sia. Though no literature is currently available that suggests that the presence of any of the mentioned intermediates, and/or the final product could be detrimental to a non-human host, such as a fungal host, a preferred embodiment of the invention has one or more of the enzymes under the control of a regulatable (e.g., an inducible) promoter. In this embodiment, the gene (or ORF) encoding the protein of interest (including but not limited to: UDP-GlcNAc 2-epimerase/ManNAc kinase, *NeuC,* and GlcNAc epimerase) is cloned downstream of an inducible promoter (including but not limited to: the alcohol oxidase promoter (AOX1 or AOX2; Tschopp, J.F., Brust, P.F., et al. 1987), galactose-inducible promoter (GAL10; Yocum, R.R., Hanley, S., et al. 1984), tetracycline-inducible promoter (TET; Belli, G., Gari, E., et al. 1998)) to facilitate the controlled expression of that enzyme, and thus regulate the production of CMP-Sia.

### Detection Of CMP-Sialic Acid And The Intermediate Compounds In Its Synthesis

The methods of the present invention provide engineered pathways to produce a cellular pool of CMP-Sia in non-human host cells which lack an endogenous CMP-Sia biosynthetic pathway. To assess the production of each intermediate in the pathway, these intermediates must be detectable. Accordingly, the present invention also provides a method for detecting such intermediates. A method for detecting a cellular pool of CMP-Sia, for example, is provided in **Example 10**. Currently, the literature describes only a few methods for measuring cellular CMP-Sia and its precursors. Early methods involved paper chromatography and thiobarbituric acid analysis and were found to be complicated and time consuming (Briles, E.B., Li, E., et al. 1977) (Harms, E., Kreisel, W., et al. 1973). HPLC (high pressure liquid chromatography) has also been used, though earlier methods employed acid elution resulting in the rapid hydrolysis of the CMP-Sia (Rump, J.A., Phillips, J., et al. 1986). Most recently, a more robust method has been described using high-performance anion-exchange chromatography using an alkaline elution protocol combined with pulsed amperometric detection (HPAEC-PAD) (Fritsch, M., Geilen, C.C., et al. 1996). This method, in addition to detecting CMP-Sia, can also detect the precursor sialic acid, thus being useful for confirming cellular synthesis of either or both of these compounds.

### Codon Optimization And Nucleotide Substitution

The methods discloded herein may be performed in conjunction with optimization of the base composition for efficient transcription/translation of the encoded protein in a particular host, such as a fungal host For example, because the Neu genes introduced into a fungal host are of bacterial origin, it may be necessary to optimize the base pair composition. This includes codon optimization to ensure that the cellular pools of tRNA are sufficient The foreign genes (ORFs) may contain motifs detrimental to complete transcription/translation in the fungal host and, thus, may require substitution to more amenable sequences. The expression of each introduced protein can be followed both at the transcriptional and translational stages by well known Northern and Western blotting techniques, respectively (Sambrook, J. and Russell, D.W., 2001).

### Vectors

In another aspect, the present disclosure provides vectors (including expression vectors), comprising genes encoding activities which promote the CMP-Sia biosynthetic pathway, a promoter, a terminator, a selectable marker and targeting flanking regions. Such promoters, terminators, selectable markers and flanking regions are readily available in the art. In a preferred embodiment, the promoter in each case is selected to provide optimal expression of the protein encoded by that particular ORF to allow sufficient catalysis of the desired enzymatic reaction. This step requires choosing a promoter that is either constitutive or inducible, and provides regulated levels of transcription. In another embodiment, the terminator selected enables sufficient termination of transcription. In yet another embodiment, the selectable markers used are unique to each ORF to enable the subsequent selection of a fungal strain that contains a specific combination of the ORFs to be introduced. In a fiuther embodiment, the locus to which each fusion construct (encoding promoter, ORF and terminator) is localized, is determined by the choice of flanking region.

### Integration Sites

The integration of multiple genes into the chromosome of the host cell is likely required and involves a thoughtful strategy. The engineered strains are transformed with a range of different genes, and these genes are transformed in a stable fashion to ensure that the desired activity is maintained throughout the fermentation process. Any combination of the previously mentioned enzyme activities will have to be engineered into the host In addition, a number of genes which encode enzymes known to be characteristic of non-human glycosylation reactions will need to be deleted from the non-human host cell. Genes which encode enzymes known to be characteristic of non-human glycosylation reactions in fugal hosts and their corresponding proteins have been extensively characterized in a number of lower eukaryotes (e.g., *Saccharomyces cerevisiae, Trichoderma reesei, Aspergillus nidulans, P. pastoris,* etc.), thereby providing a list of known glycosyltransferases in lower eukaryotes, their activities and their respective genetic sequence. These genes are likely to be selected from the group of mannosyltransferases e.g., 1,3 mannosyltransferases (e.g., *MNN1* in *S*. *cerevisiae*) (Graham, T. and Emr, S. 1991), 1,2 mannosyltransferases (e.g., the KTR/KRE family from *S*. *cerevisiae),* 1,6 mannosyltransferases (*OCH1* from *S*. *cerevisiae),* mannosylphosphate transferases and their regulators (*MNN4* and *MNN6* from *S*. *cerevisiae)* and additional enzymes that are involved in aberrant (i.e. non-human) glycosylation reactions.

Genes that encode enzymes that are undesirable serve as potential integration sites for genes that are desirable. For example, 1,6 mannosyltransferase activity is a hallmark of glycosylation in many known lower eukaryotes. The gene encoding α-1,6 mannosyltransferase (*OCH1*) has been cloned from *S. cerevisiae* (Chiba et al., 1998) as well as the initiating 1,6 mannosyltransferase activity in *P*. *pastoris* (WO 02/00879) and mutations in the gene produce a viable phenotype with reduced mannosylation. The gene locus encoding α-1,6 mannosyltransferase activity is, therefore, a prime target for the integration of genes encoding glycosyltransferase activity. Similarly, one can choose a range of other chromosomal integration sites resulting in a gene disruption event that is expected to: (1) improve the cells ability to glycosylate in a more human-like fashion, (2) improve the cells ability to secrete proteins, (3) reduce proteolysis of foreign proteins and (4) improve other characteristics of the process that facilitate purification or the fermentation process itself.

### Host Cell Production Strain

A host cell which lacks an endogenous CN2-Sia biosynthetic pathway and which expresses a functional CMP- Sia biosynthetic pathway is provided. In one embodiment, a fungal host cell which expresses a functional CMP-Sia biosynthetic pathway is provided. Preferably, the host produces a cellular pool of CMP-Sia that may be used as a donor molecule in the presence of a sialyltransferase and a glycan acceptor (e.g., Gal₂GlcNAc₂Man₃GlcNAc₂) in a sialylation reaction. Using the methods disclosed herein, a variety of different hosts producing CMP-Sia may be generated. Preferably, robust protein production strains of fungal hosts that are capable of performing well in an industrial fermentation process are selected. These strains, which produce acceptor glycans, for example, that are galactosylated includes, without limitation: *Pichia pastoris, Pichia finlandica, Pichia trehalophila, Pichia koclamae, Pichia membranaefaciens, Pichia minuta* (*Ogataea minuta, Pichia lindneri*), *Pichia opuntiae, Pichia thermotolerans, Pichia salictaria, Pichia guercuum, Pichia pijperi, Pichia stiptis, Pichia methanolica, Pichia sp., Saccharomyces cerevisiae, Saccharomyces sp., Hansenula polymorpha, Kluyveromyces sp., KZuyveromyces lactis, Candida albicans, Aspergillus ni dulans, Aspergillus niger, Aspergillus oryzae, Trichoderma reesei, Chrysosporium lucknowense, Fusarium sp., Fusarium gramineum, Fusarium venenatum* and *Neurospora crassa.* Preferably, the modified strains of the present invention are used to produce human-like sialylated glycoproteins according to the methods provided in WO 02/00879, WO 03/056914 and US2004/0018590.

### Therapeutic Proteins

The fungal host strains produced according to methods of the present disclosure combined with the teachings described in WO 02/00879, WO 03/056914 and US2004/0018590, produce high titers of heterologous therapeutic proteins in which a wide variety of sialylated glycans on a protein of interest, such as a recombinant protein, is generated in a host which lacks endogenous CMP-Sia, such as a fungal host, including without limitation: erythropoietin, cytokines such as interferon-α interferon-β, interferon-γ, interferon-ω, TNTF-α granulocyte-CSF, GM-CSF, interleukins such as IL-1ra, coagulation factors such as factor VIII, factor IX, human protein C, antithrombin III and thrombopoeitin, antibodies; IgG, IgA, IgD, IgE, IgM and fragments thereof, Fc and Fab regions, soluble IgE receptor α-chain, urokinase, chymase, and urea trypsin inhibitor, IGF-binding protein, epidermal growth factor, growth hormone-releasing factor, FSH, annexin V fusion protein, angiostatin, vascular endothelial growth factor-2, myeloid progenitor inhibitory factor-1, osteoprotegerin, α-1 antitrypsin, DNase II, α-feto proteins and glucocerebrosidase. These and other sialylated glycoproteins are particularly useful for therapeutic administration.

The following are examples which illustrate the compositions and methods of this invention.

### EXAMPLE 1

### Cloning Enzymes Involved In CMP-Sialic Acid Synthesis

One method for cloning a CMP-sialic acid biosynthetic pathway into a fungal host cell involves amplifying the *E*. *coli NeuA, NeuB* and *NeuC* genes from *E. coli* genomic DNA using the polymerase chain reaction in conjunction with primer pairs specific for each open reading frame (ORF) (**Table 1**, below and **Figures 4****,** **3** and **2****,** respectively).

For cloning a mammalian CMP-sialic acid biosynthetic pathway, the mouse CMP-Sia synthase ORF (**Figure 5**) was amplified from a mouse pituitary cDNA library in conjunction with the primer pairs set forth in **Table 1.** The GlcNAc epimerase (previously discussed in an alternate method for producing CMP-Sia intermediates), was amplified from porcine DNA using PCR in conjunction with primer pairs specific for the corresponding gene (**Table 1** and **Figure 7**). The sialate aldolase gene (**Figure 9**) was amplified from *E*. *coli* genomic DNA using the polymerase chain reaction in conjunction with the primer pairs set forth in **Table 1.** The mouse bifunctional UDP-N-acetylglucosamine-2-Epimerase/N-acetylmannosamine kinase gene was amplified from mouse liver using the polymerase chain reaction in conjunction with the primer pairs set forth in **Table 1.** The mouse N-acetylneuraminate-9-phosphate synthase gene was amplified from mouse liver using the polymerase chain reaction in conjunction with the primer pairs set forth in **Table 1.** The human CMP-Sia synthase gene was amplified from human liver using the polymerase chain reaction in conjunction with the primer pairs set forth in **Table 1.** In each case, the ORFs were amplified using a high-fidelity DNA polymerase enzyme under the following thermal cycling conditions: 97°C for 1 min, 1 cycle; 97°C for 20 sec, 60°C for 30 sec, 72°C for 2 min, 25 cycles; 72°C for 2min, 1 cycle. Following DNA sequencing to confirm the absence of mutations, each ORF is re-amplified using primers containing compatible restriction sites to facilitate the subcloning of each. into suitable fungal expression vectors.

**Table 1:**

| **Primer name** | **Primer sequence** |
|---|---|
| *Neu*A sense | 5'- ATGAGAACAAAAATTATTGCGAT[?]AATTCCAGCCCG- 3' (SEQ ID NO:1) |
| *NeuA* antisense | 5'-TCATTTAACAATCTCCGCTATTTCGTTTTC-3' (SEQ ID NO:2) |
| *NeuB* sense | 5'- ATGAGTAATATATATATATCGTTGCT`GAAATTGGTTG- 3' (SEQ ID NO:3) |
| *NeuB* antisense | 5'-TTATTCCCCCTGATTTTTGAATT CGCTATG-3' (SEQ ID NO:4) |
| *NeuC* sense | 5'- ATGAAAAAAATATTATACGTAACTGGATCTAGAG-3' (SEQ ID NO:5) |
| *NeuC* antisense | 5'-CTAGTCATAACTGGTGGTACATTCCGGGATGTC-3' (SEQ ID NO:6) |
| mouse CMP-Sia synthase sense | 5'-ATGGACGCGCTGGAGAAGGGGCGCCGTCACGTC-3' (SEQ ID NO:7) |
| mouse CMP-Sia synthase antisense | 5'- CTATTTTTGGCATGAGTTATTAACTTTTTCTATCAG- 3' (SEQ ID NO:8) |
| porcine ClcNAc epimerase sense | 5'-ATGGAGAAGGAGCGCGAAACTCTGCAGG-3' (SEQ ID NO:9) |
| porcine GlcNAc epimerase antisense | 5'-CTAGGCGAGGCGGCTCAGCAGGGCGCTC-3' (SEQ ID NO:10) |
| *E. coli* Sialate aldolase sense | 5'-ATGGCAACGAATTTACGTGGCGTAATGGCTG-3' (SEQ ID NO:11) |
| *E. coli* Sialate aldolase antisense | 5'-TCACCCGCGCTCTTGCATCAACTGCTGGGC-3' (SEQ ID NO:12) |
| mouse bifunctional UDP-N-acetylglucosamine-2-epimerase/N-acetylmannosamine kinase sense | 5'-ATGGAGAAGAACGGAACAACCGAAAGCTCCG-3' (SEQ ID NO:25) |
| mouse bifunctional UDP-N-acetylglucosamine-2-epimerase/N-acetylmannosamine kinase antisense | 5'-CTAGTGGATCCTGCGCGTTGTGTAGTCCAG-3' (SEQ ID NO:26) |
| mouse Sia9P syn sense | 5'-ATGCCGCTGGAACTGGAGCTGTGTCCCGGGC-3' (SEQ ID NO:27) |
| mouse Sia9P syn antisense | 5'-TTAAGCCTTGATTTTCTTGCTGTGACTTTCCAC -3' (SEQ ID NO:28) |
| human CMP-Sia synthase sense | 5'-ATGGACTCGGTGGAGAAGGGGGCCGCCACC-3' (SEQ ID NO:29) |
| human CMP-Sia synthase antisense | 5'-CTATTTTTGGCATGAATTATTAACTTTTTCC-3' (SEQ ID NO:30) |

### EXAMPLE 2

### Expression Of Bacterial Neu Genes In P. pastoris

The 1176 bp PCR amplified fragment of the *NeuC* gene was ligated into the *NotI-AscI* site in the yeast integration vector **pJN348** (a modified pUC 19 vector comprising a GAPDH promoter, a *NotI AscI PacI* restriction site cassette, CycII transcriptional terminator, URA3 as a positive selection marker) producing **pSH256.** Similarly, the PCR amplified fragment (1041 bp) of the *NeuB* gene was ligated into the *NotI-PacI* site in the yeast integration vector **pJN335** under a GAPDH promoter using ADE as a positive selection marker producing **pSH255.** The 1260 bp PCR amplified fragment of the *NeuA* gene was ligated into the *NotI-PacI* site in the yeast integration vector **pJN346** under a GAPDH promoter with ARG as a positive selection marker to produce **pSH254.** After transforming *P. pastoris* with each vector by electroporation, the cells were plated onto the corresponding drop-out agar plates to facilitate positive selection of the newly introduced vector(s). To confirm the introduction of each gene, several hundred clones were repatched onto the respective dropout plates and grown for two days at 26°C. Once sufficient material had grown, each clone was screened by colony PCR using primers specific for the introduced gene. Conditions for colony PCR using the polymerase ExTaq from Takara, were as follows: 97°C for 3 min, 1 cycle; 97°C for 20 sec, 50°C for 30 sec, 72°C for 2 min/kb, 30 cycles; 72°C for 10 min, 1 cycle. Subsequently, several positive clones from colony PCR were grown in a baffled flask containing 200 ml of growth media. The base composition of growth media containing 2.68 g/l yeast nitrogen base, 200 mg/l biotin and 2 g/l dextrose was supplemented with amino acids depending on the strain used. The cells were grown in this media in the presence or absence of 20 mM ManNAc. Following growth in the baffle flask at 30°C for 4-6 days, the cells were pelleted and analyzed for intermediates of the sialic acid pathway, as described in **Example 10.**

### EXAMPLE 3

### Expression of GlcNAc Epimerase Gene In P. pastoris

The PCR amplified fragment of the porcine GlcNAc epimerase gene was ligated into the *NotI-PacI* site in the yeast integration vector **pJN348** under the control of the GAPDH promoter, using URA3 as a positive selection marker. The *P. pastoris* strain producing endogenous GlcNAc was transformed with the vector carrying the GlcNAc epimerase gene fragment and screened for transformants.

### EXAMPLE 4

### Expression of Sialate Aldolase Gene In P. pastoris

The PCR amplified fragment of the *E. coli* sialate aldolase gene was ligated into the *NotI-PacI* site in the yeast integration vector **pJN335** under the control of the GAPDH promoter with ADE as a positive selection marker producing **pSH275.** The *P. pastoris* strain producing ManNAc was transformed with the vector carrying the sialate aldolase gene fragment and screened for transformants.

### EXAMPLE 5

### Expression Of The Gene Encoding UDP-N-acetylglucosamine-2-Epimerase/N-acetylmannosamine Kinase in P. pastoris

The PCR amplified fragment of the gene encoding the mouse bifunctional UDP-N-acetylglucosamine-2-Epimerase/N-acetylmannosamine Kinase enzyme was ligated into the *NotI-PacI* site in the yeast integration vector **pJN348** under the control of the GAPDH promoter with URA as a positive selection marker producing **pSH284.** The *P. pastoris* strain producing ManNAc was transformed with the vector carrying the gene fragment and screened for transformants.

### EXAMPLE 6

### Expression Of The Gene Encoding N-acetyl-neuraminate-9-Phosphate Synthase In P. pastoris

The PCR amplified fragment of the mouse N-acetylneuraminate-9-phosphate synthase gene was ligated into the *NotI-PacI* site in the yeast integration vector **pJN335** under the control of the GAPDH promoter with ADE as a positive selection marker producing **pSH285.** The *P. pastoris* strain producing ManNAc-6-P was transformed with the vector carrying the above gene fragment and screened for transformants.

### EXAMPLE 7

### Identification, Cloning And Expression Of The Gene Encoding N-acetylneuraminate-9-Phosohatase

N-acetylneuraminate-9-phosphatase activity has been detected in the cytosolic fraction of rat liver cells (Van Rinsum, J., Van Dijk, W. 1984). We have repeated this method and isolated a cell extract fraction containing phosphatase activity only against NeuAc-9-P. SDS-PAGE electrophoresis of this fraction identifies a single protein band. Subsequently, this sample was electroblotted onto a PDVF membrane, and the N-terminal amino acid sequence was identified by Edman degradation. The sequence identified allows the generation of degenerate oligonucleotides for the 5'-terminus of the ORF of the isolated protein. Using these degenerate primers in conjunction with the AP1 primer supplied in a rat liver Marathon-ready cDNA library (Clontech), a full length ORF was isolated according to the manufacturer's instructions. The complete ORF was subsequently ligated into the yeast integration vector **pJN347** (WO 02/00879) under the control of the GAPDH promoter with a HIS gene as a positive selection marker. The *P. pastoris* strain producing NeuAc-9-P was transformed with the vector carrying the desired gene fragment and screened for transformants as described in **Example 2.**

### EXAMPLE 8

### Cloning And Expression Of A CMP-Sialic Acid Synthase Gene In P.pastoris

The PCR amplified fragment of the mouse CMP-Sia synthase gene was ligated into the NotI-PacI site in the yeast integration vector **pJN346** under the control of the GAPDH promoter with the ARG gene as a positive selection marker. A *P. pastoris* strain producing sialic acid was transformed with the vector carrying the above gene fragment and screened for transformants as described **Example 2**. Likewise, the human CMP-Sia synthase gene (Genbank: AF397212) was amplified and ligated into the *NotI-PacI* site of the yeast expression vector **pJN346** producing the vector **pSH257.** A *P. pastoris* strain capable of producing sialic acid was transformed with **pSH257** by electroporation, producing a strain capable of generating CMP-Sia.

### EXAMPLE 9

### Expression Of The Hybrid CMP-Sia Pathway In P. pastoris

The *P. pastoris* strain **JC308** (Cereghino, 2001 Gene 263, 159-164) was super-transformed with 20 mg of each of the vectors containing *NeuC* (**pSH256**), *NeuB* (**pSH255**) and hCMP-Sia synthase (**pSH257**) by electroporation. The resultant cells were plated on minimal media supplemented with histidine (containing 1.34 g/l yeast nitrogen base, 200 mg/l biotin, 2 g/l dextrose, 20 g/l agar and 20 mg/l L-histidine). Following incubation at 30°C for 4 days, several hundred clones were isolated by repatching onto minimal media plates supplemented with histidine (see above for composition). The repatched clones were grown for 2 days prior to performing colony PCR (as described in **Example 2**) on the clones. Primers specific for *NeuC, NeuB* and hCMP-Sia synthase were used to confirm the presence of each ORF in the transformed clones. Twelve clones positive for all three ORFs (designated **YSH99a-I**) were grown in a baffled flask containing 200 ml of growth media (containing 2.68 g/l yeast nitrogen base, 200 mg/l biotin, 20 mg/l L-histidine and 2 g/l dextrose). The effect of supplementing the growth media with ManNAc was investigated by growing the cells in the presence or absence of 20 mM ManNAc. Following growth in the baffle flask at 30°C for 4-6 days the cells are pelleted and analyzed for the presence of sialic acid pathway intermediates as described in **Example 10.**

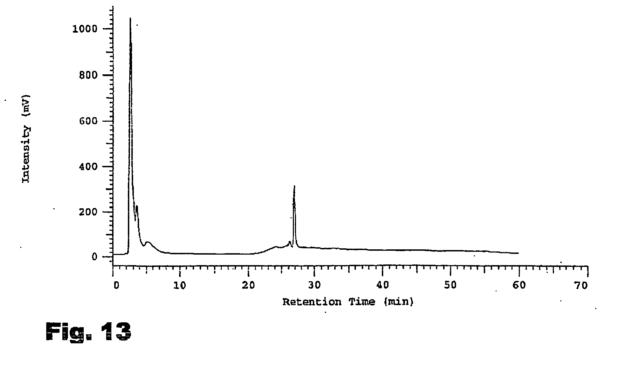

Comparing the cell extracts using the assay outlined in **Example 10,** the cell extracts from *P. pastoris* **YSH99a** without exogenous CMP-Sia, showed transfer of Sia onto acceptor substrates indicating the presence of CMP-Sia (**Figure 12**). Both mono- and di-sialylated biantennary N-glycans eluted at 20 min and 23 min, their respective corresponding time. Additionally, the sialidase treatment (**Example 11**) showed the removal of sialic acid (**Figure 13**). Thus, a yeast strain engineered with a hybrid CMP-Sia biosynthetic pathway as described, containing the *NeuC, NeuB* and hCMP-Sia synthase, is capable of generating an endogenous pool of CMP-sialic acid.

### EXAMPLE 10

### Assay For The Presence of Cytidine-5'-Monophospho-N-Acetylneuraminic Acid In Genetically Altered P.pastoris

Yeast cells were washed three times with cold PBS buffer, and suspended in 100 mM ammonium bicarbonate pH 8.5 and kept on ice. The cells were lysed using a French pressure cell followed by sonication. Soluble cell contents were separated from cell debris by ultracentrifugation. Ice cold ethanol was added to the supernatant to a final concentration of 60% and kept on ice for 15 minutes prior to removal of insoluble proteins by ultracentrifugation. The supernatant was frozen and concentrated by lyophilization. The dried sample was resuspended in water (ensuring pH is 8.0) and then filtered through a pre-rinsed 10,000 MWCO Centricon cartridge. The filtrate was separated on a Mono Q ion-exchange column and the elution fractions that co-elute with authentic CMP-sialic acid are pooled and lyophilized.

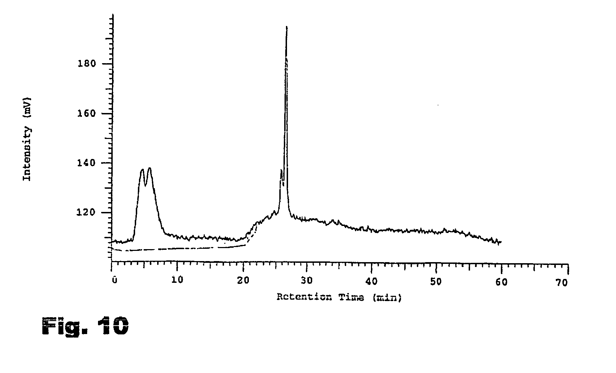

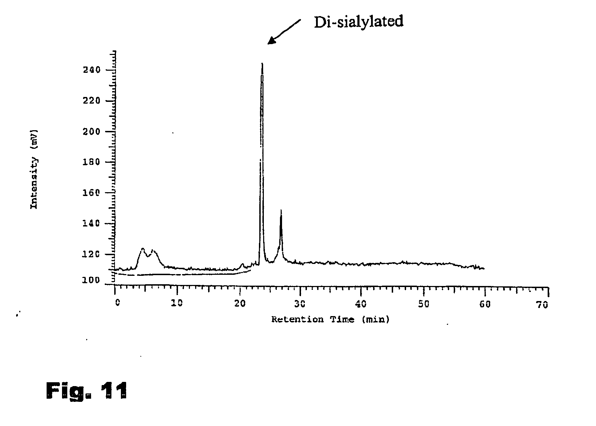

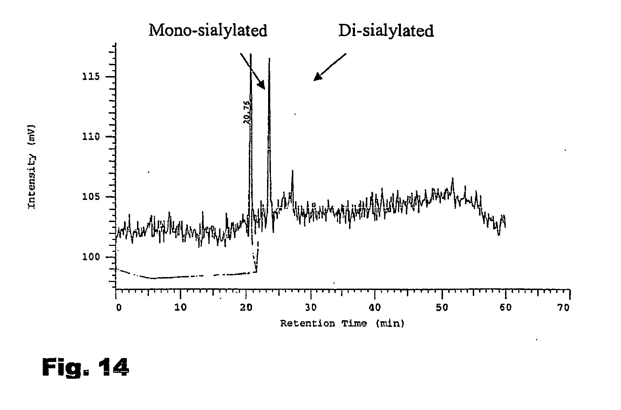

The dried filtrate was dissolved in 100 *µ*L of 100 mM ammonium acetate pH6.5, 11 *µ*L (5 mU) of α-2,6 sialyltransferase and 3.3 *µ*L(12 mU) of α-2,3 sialyltransferase were added, and 10 *µ*L of the mixture was removed for a negative control. Subsequently, 7 *µ*L (1.4 *µ*g) of 2-aminobenzamide-labeled asialo-biantennary N-glycan (NA2, Glyco Inc., San Rafael, CA) was added to the remaining mixture, followed by the removal of 10 *µ*L for a positive control. The sample and control reactions were then incubated at 37°C for 16 hr. 10*µ*L of each sample were then separated on a GlycoSep-C anion exchange column according to manufacturer's instructions. A separate control consisting of approximately 0.05 *µ*g each of monosialylated and disialylated biantennary glycans was separated on the column to establish relative retention times. The results are shown in **Figures 10-14**.

### EXAMPLE 11

### Sialidase Treatment

The incubation of bi-antennary galactosylated N-glycans with an extract from the *P. pastoris* **YSH99a** strain in the presence of sialyltransferases produced sialylated N-glycans, which were subsequently desialylated as follows: a sialylated sample was passed through a Microcon cartridge, with 10,000 molecular weight cut-off, to remove the transferases. The cartridge was washed twice with 100 *µ*l of water, which was pooled with the original eluate. Analysis of the eluate by HPLC (**Figure 13**) produced a spectrum similar to the HPLC spectrum prior to the Microcon treatment. The remaining sample was lyophilized to dryness and resuspended in 25 *µ*l of 1xNEB G1 buffer. After addition of 100 U of sialidase (New England Biolabs #P0720L, Beverley, MA), the resuspended sample was incubated overnight at 37°C prior to HPLC analysis, as described previously.

### REFERENCES:

Alviano, C.S., Travassos, L.R., et al. (1999) Sialic acids in fungi: A minireview. Glycoconjugate Journal, 16, 545-554.
Annunziato, P.W., Wright, L.F., et al. (1995) Nucleotide sequence and genetic analysis of the neuD and neuB genes in region 2 of the polysialic acid gene cluster of Escherichia coli K1. J. Bacteriol., 177, 312-319.
Ballou, C.E. (1990) Isolation, characterization, and properties of Saccharomyces cerevisiae mnn mutants with nonconditional protein glycosylation defects. Methods Enzymology, 185, 440-470.
Belli, G., Gari, E. et al. (1998) An activator/ repressor dual system allows tight tetracycline-regulated gene expression in budding yeast. Nucleic Acids Res., 26, 942-947.
Briles, E.B., Li, E., et al. (1977) Isolation of wheat germ agglutinin-resistant clones of Chinese hamster ovary cells deficient in membrane sialic acid and galactose. J. Biol. Chem., 252, 1107-1116.
Chiba, Y., Suzuki, M., et al. (1998) Production of human compatible high mannose-type (Man(5)GlcNAc(2)) sugar chains in Saccharomyces cerevisiae. Journal of Biological Chemistry, 273, 26298-26304.
Choi, B.K., Bobrowicz, P. et al. (2003) Use of combinatorial genetic libraries to humanize N-linked glycosylation in the yeast Pichia pastoris. Proc. Nat'l Acad. Sci. USA. Apr 29;100(9):5022-7.
Cregg, J.M. et al. (2000). Recombinant protein expression in Pichia pastoris. Mol. Technol., 16, 23-52.
Fritsch, M., Geilen, C.C., et al. (1996) Determination of cytidine 5'-monophospho-N-acetylneuraminic acid pool size in cell culture scale using high-performance anion-exchange chromatography with pulsed amperometric detection. J. Chromatogr. A., 727, 223-230.
Fukuda, M.N., Sasaki, H., et al. (1989) Survival of recombinant erythropoietin in the circulation: the role of carbohydrates. Blood, 73, 84-89.
Graham, T. and Emr, S. (1991) Compartmental organization of Golgi-specific protein modification and vacuolar protein sorting events defined in a yeast sec 18 (NSF) mutant. J. Cell. Biol., 114, 207-218.
Hamilton, S.R., Bobrowicz, P., et al. (2003) Production of Complex Human Glycoproteins in Yeast. Science, 301, 1244-1246.
Harms, E., Kreisel, W., et al. (1973) Biosynthesis of N-acetylneuraminic acid in Morris hepatomas. Eur. J. Biochem., 32, 254-262.
Hinderlich, S., Stasche, R., et al. (1997) A bifunctional enzyme catalyzes the first two steps in N-acetylneuraminic acid biosynthesis of rat liver. Purification and characterization of UDP-N-acetylglucosamine 2-epimerase/N-acetylmannosamine kinase. J. Biol. Chem., 272, 24313-24318.
Inoue, H., Fukui, K., et al. (1990) Molecular cloning and sequence analysis of a cDNA encoding a porcine kidney renin-binding protein. J. Biol. Chem., 265, 6556-6561.
Kelm, S. and Schauer, R. (1997) Sialic acids in molecular and cellular interactions. Int. Rev. Cytol., 175,137-240.
Keppler, O.T., Hinderlich, S. et al. (1999) UDP-GlcNAc 2-epimerase: A regulator of cell surface sialylation. Science, 284, 1372-1376.
Lawrence, S.M., Huddleston, K.A., et al. (2000) Cloning and expression of the human N-acetylneuraminic acid phosphate synthase gene with 2-keto-3-deoxy-D-glycero- D-galacto-nononic acid biosynthetic ability. J. Biol. Chem., 275, 17869-17877.
Lin Cereghino, G.P., Lin Cereghino, J., et al. (2001) New selectable marker/auxotrophic host strain combinations for molecular genetic manipulation of Pichia pastoris. Gene, 263, 159-169.
MacDougall, I.C., Gray, S.J., et al. (1999). Pharmacokinetics of Novel Erythropoeisis Stimulating Protein Compared with Epoetin Alfa in Dialysis Patients. J. Am. Soc. Nephrol. 10, 2392-2395.
Maru, I., Ohta, Y., Murata, et al. (1996) Molecular cloning and identification of N-acyl-D-glucosamine 2-epimerase from porcine kidney as a renin-binding protein. J. Biol. Chem., 271,16294-16299.
Munster, A.K., Eckhardt, M., et al. (1998) Mammalian cytidine 5'-monophosphate N-acetylneuraminic acid synthetase: a nuclear protein with evolutionarily conserved structural motifs. Proc. Nat'l Acad. Sci. USA, 95, 9140-9145.
Nakanishi-Shindo, Y., Nakayama, K., et al. (1993) Structure of the N-Linked Oligosaccharides That Show the Complete Loss of Alpha-1,6-Polymannose Outer Chain From Och1, Och1 Mnnl, and Och1 Mnnl Alg3 Mutants of Saccharomyces-Cerevisiae. J. Biol. Chem., 268, 26338-26345.
Ohta, Y., Watanabe, K. et al. (1985) Complete nucleotide sequence of the E. coli N-acetylneuraminate lyase. Nucleic Acids Res. 13, 8843-8852.
Parodi, A.J. (1993) N-glycosylation in trypanosomatid protozoa. Glycobiology, 3, 193-199.
Ringenberg, M., Lichtensteiger, C., et al. (2001) Redirection of sialic acid metabolism in genetically engineered Escherichia coli. Glycobiology, 11, 533-539.
Rump, J.A., Phillips, J., et al. (1986) Biosynthesis of gangliosides in primary cultures of rat hepatocytes. Determination of the net synthesis of individual gangliosides by incorporation of labeled N-acetylmannosamine. Biol. Chem. Hoppe Seyler, 367, 425-432.
Sambrook, J. and Russell, D.W. (2001) Molecular Cloning: A laboratory manual. 3rd Edition. Cold Spring Harbor Laboratory Press, Cold Spring Harbor NY. Schauer, R. (2000. Achievements and challenges of sialic acid research. Glycoconj. J. 17, 485-99.
Spivak, J.L. and Hogans, B.B. (1989) The in vivo metabolism of recombinant human erythropoietin in the rat. Blood, 73, 90-99.
Stasche, R., Hinderlich, S., et al. (1997) A bifunctional enzyme catalyzes the first two steps in N-acetylneuraminic acid biosynthesis of rat liver. Molecular cloning and functional expression of UDP-N-acetyl-glucosamine 2-epimerase/N-acetylmannosamine kinase. J. Biol. Chem., 272, 24319-24324.
Tschopp, J.F., Brust, P.F., et al. (1987) Expression of the lacZ gene from two methanol-regulated promoters in Pichia pastoris. Nucleic Acids Res. 15, 3859-3876.
Van Rinsum, J., Van Dijk, W., et al. (1984) Subcellular localization and tissue distribution of sialic acid forming enzymes. Biochem. J., 223, 323-328.
Vimr, E., Steenbergen, S., et al. (1995) Biosynthesis of the polysialic acid capsule in Escherichia coli K1. J. Ind. Microbiol., 15, 352-360.
Vimr, E.R. and Troy, F.A. (1985) Regulation of sialic acid metabolism in Escherichia coli: Role of N-acylneuraminate pyruvate-lyase. J. Bacteriol. 164, 854-860.
Warren, L. (1994) Bound Carbohydrates in Nature. Cambridge University Press, Cambridge, U.K.
Yocum, R.R., Hanley, S. et al. (1984) Use of lacZ fusions to delimit regulatory elements of the inducible divergent GAL1-GAL10 promoter in Saccharomyces cerevisiae. Mol. Cell. Biol., 4, 1985-1998.
Yoko-o, T., Tsukahara, K., et al. (2001) Schizosaccharomyces pombe och1(+) encodes alpha-1,6-mannosyltransferase that is involved in outer chain elongation of N-linked oligosaccharides. FEBS Lett, 489, 75-80.
Zapata, G., Crowley, J.M., et al. (1992) Sequence and expression of the Escherichia coli K1 neuC gene product. J. Bacteriol., 174, 315-319.
Zapata, G., Vann, W.F., et al. (1989) Sequence of the cloned Escherichia coli K1 CMP-N-acetylneuraminic acid synthetase gene. J. Biol. Chem., 264, 14769-14774.

The disclosure also comprises the following items:
1. A recombinant non-human host cell comprising a cellular pool of CMP-Sia, wherein said host cell lacks CMP-Sia.
2. A recombinant fungal host cell comprising a cellular pool of CMP-Sia.
3. A recombinant fungal host cell comprising a CMP-Sia biosynthetic pathway.
4. A recombinant fungal host cell comprising one or more enzymes that participate in the biosynthesis of CMP-Sia.
5. The recombinant fungal host cell of any one of items 1-4, comprising a cellular pool of CMP-Sia.
6. The recombinant fungal host cell of any one of items 1 -4, wherein the CMP-Sia comprises a sialic acid selected from NeuSAc, N-glycolylneuraminic acid (Neu5Gc), and keto-3-deoxy-D-glycero-D-galacto-nononic acid (KDN).
7. The recombinant fungal host cell of any one of items 1-4, wherein the host cell expresses one or more enzyme activities selected from *E*. *coli NeuC, E. coli NeuB* and a mammalian CMP-sialate synthase activity.
8. The recombinant fungal host cell of any one of items 1-4, wherein the host cell expresses at least one enzyme activity selected from UDP-GlcNAc epimerase, sialate synthase, CMP-sialate synthase, UDP-N-acetylgluco-samine-2-epimerase, N-acetylmannosamine kinase, N-acetylneuraminate-9-phosphate synthase, N-acetylneuraminate-9-phosphatase and CMP-sialic acid synthase.
9. The recombinant fungal host cell of any one of items 1-4, wherein the host is selected from the group consisting of *Pichia pastoris, Pichia finlandica, Pichia trehalophila, Pichia koclamae, Pichia membranaefaciens, Pichia minuta, Ogataea minuta, Pichia lindneri, Pichia opuntiae, Pichia thermotolerans, Pichia salictaria, Pichia guercuum, Pichia pijperi, Pichia stiptis, Pichia methanolica, Pichia sp., Saccharomyces cerevisiae, Saccharomyces sp., Hansenula polymorpha, Kluyveromyces sp., Kluyveromyces lactis, Candida albicans, Aspergillus nidulans, Aspergillus niger, Aspergillus oryzae, Aspergillus sp, Trichoderma reesei, Chrysosporium lucknowense, Fusarium sp., Fusarium gramineum, Fusarium venenatum* and *Neurospora crassa.*
10. The recombinant fungal host cell of any one of items 1-4, wherein said host cell produces at least one intermediate selected from the group consisting of UDP-GlcNAc, ManNAc, ManNAc-6-P, Sia-9-P and Sia.
11. The recombinant fungal host cell of any one of items 1-4, wherein said host cell expresses a heterologous therapeutic protein selected from the group consisting of: erythropoietin, cytokines, interferon-α, interferon-β, interferon-γ, interferon-ω, TNF-α, granulocyte-CSF, GM-CSF, interleukins, IL-1ra, coagulation factors, factor VIII, factor IX, human protein C, antithrombin III and thrombopoeitin, IgA antibodies or fragments thereof, IgG antibodies or fragments thereof, IgA antibodies or fragments thereof, IgD antibodies or fragments thereof, IgE antibodies or fragments thereof, IgM antibodies and fragments thereof, soluble IgE receptor α-chain, urokinase, chymase, urea trypsin inhibitor, IGF-binding protein, epidermal growth factor, growth hormone-releasing factor, FSH, annexin V fusion protein, angiostatin, vascular endothelial growth factor-2, myeloid progenitor inhibitory factor-1, osteoprotegerin, α-1 antitrypsin, DNase II, α-feto proteins and glucocerebrosidase.
12. A method for producing CMP-Sia in a fungal host cell comprising expressing a CMP-Sia biosynthetic pathway.
13. The method of item 12, comprising expressing at least one enzyme activity from a prokaryotic CMP-Sia biosynthetic pathway.
14. The method of item 12, comprising expressing at least one enzyme activity from a mammalian CMP-Sia biosynthetic pathway.
15. The method of item 12, wherein said method comprises expressing a mammalian CMP-sialate synthase activity.
16. The method of item 12, comprising expressing a hybrid CMP-Sia biosynthetic pathway.
17. The method of item 12 or 16, wherein said method comprises expressing at least one enzyme activity selected from *E*. *coli NeuC, E. coli NeuB* and a mammalian CMP-sialate synthase activity.
18. The method of any one of items 12-17, wherein the host is selected from the group consisting of *Pichia pastoris, Pichia finlandica, Pichia trehalophila, Pichia koclamae, Pichia membranaefaciens, Pichia minuta, Ogataea minuta, Pichia lindneri, Pichia opuntiae, Pichia thermotolerans, Pichia salictaria, Pichia guercuum, Pichia pijperi, Pichia stiptis, Pichia methanolica, Pichia sp., Saccharomyces cerevisiae, Saccharomyces sp., Hansenula polymorpha, Kluyveromyces sp., Kluyveromyces lactis, Candida albicans, Aspergillus nidulans, Aspergillus niger, Aspergillus oryzae, Aspergillus sp, Trichoderma reesei, Chrysosporium lucknowense, Fusarium sp., Fusarium gramineum, Fusarium venenatum* and *Neurospora crassa.*
19. The method of any one of items 12-17, wherein the CMP-sialate synthase enzyme activity localizes in the nucleus of the host cell.
20. The method of claim any one of items 12-17, wherein the CMP-sialate synthesis is enhanced by supplementing a medium for growing the host cell with one or more intermediate substrates used in the CMP-Sia synthesis.
21. The method of any one of items 12-17, wherein the enzyme activity is expressed under the control of a constitutive promoter or a an inducible promoter.
22. The method of any one of items 12-17, wherein the expressed enzyme activity is from a partial ORF encoding that enzymatic activity.
23. The method of any one of items 12-17, wherein the expressed enzyme is a fusion to another protein or peptide.
24. The method of any one of items 12-17, wherein the expressed enzyme has been mutated to enhance or attenuate the enzymatic activity.
25. The method of any one of items 12-17, wherein said host cell expresses a heterologous therapeutic protein selected from the group consisting of: erythropoietin, cytokines, interferon-α, interferon-β, interferon-γ, interferon-ω, TNF-α, granulocyte-CSF, GM-CSF, interleukins, IL-1ra, coagulation factors, factor VIII, factor IX, human protein C, antithrombin III and thrombopoeitin, IgA antibodies or fragments thereof, IgG antibodies or fragments thereof, IgA antibodies or fragments thereof, IgD antibodies or fragments thereof, IgE antibodies or fragments thereof, IgM antibodies and fragments thereof, soluble IgE receptor α-chain, urokinase, chymase, urea trypsin inhibitor, IGF-binding protein, epidermal growth factor, growth hormone-releasing factor, FSH, annexin V fusion protein, angiostatin, vascular endothelial growth factor-2, myeloid progenitor inhibitory factor-1, osteoprotegerin, α-1 antitrypsin, DNase II, α -feto proteins and glucocerebrosidase,
26. A method for producing a recombinant glycoprotein comprising the step of producing a cellular pool of CMP-Sia in a fungal host and expressing said glycoprotein in said host.
27. A method for producing a recombinant glycoprotein comprising the step of engineering a CMP-Sia biosynthetic pathway in a fungal host and expressing said glycoprotein in said host.
28. The method of item 26 or 27, wherein said recombinant glycoprotein is a therapeutic protein selected from the group consisting of:
   erythropoietin, cytokines, interferon-α, interferon-β, interferon-γ, interferon-ω, TNF-α, granulocyte-CSF, GM-CSF, interleukins, IL-1ra, coagulation factors,
   factor VIII, factor IX, human protein C, antithrombin III and thrombopoeitin, IgA antibodies or fragments thereof, IgG antibodies or fragments thereof, IgA antibodies or fragments thereof, IgD antibodies or fragments thereof, IgE antibodies or fragments thereof, IgM antibodies and fragments thereof, soluble IgE receptor α-chain, urokinase, chymase, urea trypsin inhibitor, IGF-binding protein,
   epidermal growth factor, growth hormone-releasing factor, FSH, annexin V fusion protein, angiostatin, vascular endothelial growth factor-2, myeloid progenitor inhibitory factor-1, osteoprotegerin, α-1 antitrypsin, DNase II, α-feto proteins and glucocerebrosidase.

## Claims

1. A method for producing CMP-sialic acid in a yeast host cell comprising:
(a) transforming into the yeast host cell nucleic acid molecules encoding *E*. *coli* NeuC, *E*. *coli* NeuB, and a CMP-Sialic acid synthase; and
(b) growing the yeast host cell under conditions effective for producing the CMP-sialic acid.

2. The method of claim 1, wherein the yeast host cell is a *Pichia* sp., *Pichia pastoris, Pichia finlandica, Pichia trehalophila, Pichia koclamae, Pichia membranaefaciens, Pichia opuntiae, Pichia thermotolerans, Pichia salictaria, Pichia guercuum, Pichia pijperi, Pichia stiptis, Pichia methanolica; Saccharomyces* sp., *Saccharomyces cerevisiae, Hansenula polymorpha, Kluyveromyces sp., Kluyveromyces lactis,* or *Candida albicans.*

3. The method of claim 1, wherein the yeast host cell is *Pichia pastoris.*

4. A yeast host cell capable of producing CMP-sialic acid comprising:
a sialylation pathway consisting of *E*. *coli* NeuC, *E. coli* NeuB, and a CMP-Sialic acid synthase, wherein the yeast host cell is capable of producing the CMP-sialic acid.

5. The yeast host cell of claim 4, wherein the yeast host cell is a *Pichia* sp., *Pichia pastoris, Pichia finlandica, Pichia trehalophila, Pichia koclamae, Pichia membranaefaciens, Pichia opuntiae, Pichia thermotolerans, Pichia salictaria, Pichia guercuum, Pichia pijperi, Pichia stiptis, Pichia methanolica; Saccharomyces* sp., *Saccharomyces cerevisiae, Hansenula polymorpha, Kluyveromyces sp., Kluyveromyces lactis,* or *Candida albicans.*

6. The yeast host cell of claim 4, wherein the yeast host cell is *Pichia pastoris.*

7. The method of claim 1, 2 or 3, or the yeast host cell of claim 4, 5 or 6, wherein said CMP-Sialic acid synthase is a mammalian CMP-Sialic acid synthase

8. A method for producing CMP-sialic acid comprising:
(a) providing a yeast host cell comprising a CMP-sialic acid biosynthetic pathway consisting of *E. coli* NeuC, *E*. *coli* NeuB, and a CMP-Sialic acid synthase; and
(b) growing the yeast host cell under conditions effective for producing the CMP-sialic acid.

9. The method of claim 8, wherein the yeast host cell is a *Pichia* sp., *Pichia pastoris, Pichia finlandica, Pichia trehalophila, Pichia koclamae, Pichia membranaefaciens, Pichia opuntiae, Pichia thermotolerans, Pichia salictaria, Pichia guercuum, Pichia pijperi, Pichia stiptis, Pichia methanolica; Saccharomyces* sp., *Saccharomyces cerevisiae, Hansenula polymorpha, Kluyveromyces sp., Kluyveromyces lactis,* or *Candida albicans.*

10. The method of claim 8, wherein the yeast host cell is *Pichia pastoris.*

## Patentansprüche

1. Ein Verfahren zur Produktion von CMP-Sialinsäure in einer Hefe-Wirtszelle, umfassend:
(a) Transformieren von Nukleinsäuremolekülen, die *E*. *coli* NeuC, *E. coli* NeuB und eine CMP-Sialinsäure-Synthase kodieren, in die Hefe-Wirtszelle und
(b) Kultivieren der Hefe-Wirtszelle unter Bedingungen, die die Produktion der CMP-Sialinsäure bewirken.

2. Das Verfahren nach Anspruch 1, wobei die Hefe-Wirtszelle *Pichia* sp., *Pichia pastoris, Pichia finlandica, Pichia trehalophila, Pichia koclamae, Pichia membranaefaciens, Pichia opuntiae, Pichia thermotolerans, Pichia salictaria, Pichia guercuum, Pichia pijperi, Pichia stiptis, Pichia methanolica; Saccharomyces* sp., *Saccharomyces cerivisiae, Hansenula polymorpha, Kluyveromyces sp., Kluyveromyces lactis* oder *Candida albicans* ist.

3. Das Verfahren nach Anspruch 1, wobei die Hefe-Wirtszelle *Pichia pastoris* ist.

4. Eine Hefe-Wirtszelle, die in der Lage ist, CMP-Sialinsäure zu produzieren, umfassend:
einen Sialylierungsweg, bestehend aus *E*. *coli* NeuC, *E. coli* NeuB und einer CMP-Sialinsäure-Synthase, wobei die Hefe-Wirtszelle in der Lage ist, die CMP-Sialinsäure zu produzieren.

5. Die Hefe-Wirtszelle nach Anspruch 4, wobei die Hefe-Wirtszelle *Pichia* sp., *Pichia pastoris, Pichia finlandica, Pichia trehalophila, Pichia koclamae, Pichia membranaefaciens, Pichia opuntiae, Pichia thermotolerans, Pichia salictaria, Pichia guercuum, Pichia pijperi, Pichia stiptis, Pichia methanolica; Saccharomyces* sp., *Saccharomyces cerivisiae, Hansenula polymorpha, Kluyveromyces sp., Kluyveromyces lactis* oder *Candida albicans* ist.

6. Die Hefe-Wirtszelle nach Anspruch 4, wobei die Hefe-Wirtszelle *Pichia pastoris* ist.

7. Das Verfahren nach Anspruch 1, 2 oder 3 oder die Hefe-Wirtszelle nach Anspruch 4, 5 oder 6, wobei die CMP-Sialinsäure-Synthase eine Säuger-CMP-Sialinsäure-Synthase ist.

8. Ein Verfahren zur Produktion von CMP-Sialinsäure, umfassend:
(a) Bereitstellen einer Hefe-Wirtszelle, die einen CMP-Sialinsäure-Biosyntheseweg umfasst, bestehend aus *E*. *coli* NeuC, *E. coli* NeuB und einer CMP-Sialinsäure-Synthase, und
(b) Kultivieren der Hefe-Wirtszelle unter Bedingungen, die die Produktion der CMP-Sialinsäure bewirken.

9. Das Verfahren nach Anspruch 8, wobei die Hefe-Wirtszelle *Pichia* sp., *Pichia pastoris, Pichia finlandica, Pichia trehalophila, Pichia koclamae, Pichia membranaefaciens, Pichia opuntiae, Pichia thermotolerans, Pichia salictaria, Pichia guercuum, Pichia pijperi, Pichia stiptis, Pichia methanolica; Saccharomyces* sp., *Saccharomyces cerivisiae, Hansenula polymorpha, Kluyveromyces sp., Kluyveromyces lactis* oder *Candida albicans* ist.

10. Das Verfahren nach Anspruch 8, wobei die Hefe-Wirtszelle *Pichia pastoris* ist.

## Revendications

1. Procédé de production de CMP-acide sialique dans une cellule hôte de levure, comprenant:
(a) transformer dans la cellule hôte de levure, des molécules d'acide nucléique codant *E. coli* NeuC, *E. coli* NeuB et une CMP-acide sialique synthase; et
(b) cultiver la cellule hôte de levure dans des conditions efficaces pour produire du CMP-acide sialique.

2. Procédé selon la revendication 1, dans lequel la cellule hôte de levure est une *Pichia* sp., *Pichia pastoris, Pichia finlandica, Pichia trehalophila, Pichia koclamae, Pichia membranaefaciens, Pichia opuntiae, Pichia thermotolerans, Pichia salictaria, Pichia guercuum, Pichia pijperi, Pichia stiptis, Pichia methanolica; Saccharomyces* sp., *Saccharomyces cerevisiae, Hansenula polymorpha, Kluyveromyces* sp., *Kluyveromyces lactis* ou *Candida albicans.*

3. Procédé selon la revendication 1, dans lequel la cellule hôte de levure est une *Pichia pastoris.*

4. Cellule hôte de levure capable de produire du CMP-acide sialique, comprenant:
une voie de sialylation consistant en *E. coli* NeuC, *E. coli* NeuB et une CMP-acide sialique synthase, où la cellule hôte de levure est capable de produire du CMP-acide sialique.

5. Cellule hôte de levure selon la revendication 4, où la cellule hôte de levure est une *Pichia* sp., *Pichia pastoris, Pichia finlandica, Pichia trehalophila, Pichia koclamae, Pichia membranaefaciens, Pichia opuntiae, Pichia thermotolerans, Pichia salictaria, Pichia guercuum, Pichia pijperi, Pichia stiptis, Pichia methanolica; Saccharomyces* sp., *Saccharomyces cerevisiae, Hansenula polymorpha, Kluveromyces* sp., *Kluveromyces lactis* ou *Candida albicans.*

6. Cellule hôte de levure selon la revendication 4, où la cellule hôte de levure est une *Pichia pastoris.*

7. Procédé selon la revendication 1, 2 ou 3, ou bien cellule hôte de levure selon la revendication 4, 5 ou 6, où ladite CMP-acide sialique synthase est une CMP-acide sialique synthase mammalienne.

8. Procédé de production de CMP-acide sialique, comprenant:
(a) fournir une cellule hôte de levure comprenant une voie de biosynthèse de CMP-acide sialique consistant en *E. coli* NeuC, *E. coli* NeuB et une CMP-acide sialique synthase; et
(b) cultiver la cellule hôte de levure dans des conditions efficaces pour produire du CMP-acide sialique.

9. Procédé selon la revendication 8, dans lequel la cellule hôte de levure est une *Pichia* sp., *Pichia pastoris, Pichia finlandica, Pichia trehalophila, Pichia koclamae, Pichia membranaefaciens, Pichia opuntiae, Pichia thermotolerans, Pichia salictaria, Pichia guercuum, Pichia pijperi, Pichia stiptis, Pichia methanolica; Saccharomyces* sp., *Saccharomyces cerevisiae, Hansenula polymorpha, Kluveromyces* sp., *Kluveromyces lactis* ou *Candida albicans.*

10. Procédé selon la revendication 8, dans lequel la cellule hôte de levure est une *Pichia pastoris.*
